(19) Europäisches Patentamt
European Patent Office
Office européen des brevets

(11) EP 3 167 054 B1

(12) **EUROPEAN PATENT SPECIFICATION**

(45) Date of publication and mention of the grant of the patent:
**12.12.2018 Bulletin 2018/50**

(21) Application number: **15736489.4**

(22) Date of filing: **09.07.2015**

(51) Int Cl.:
***C12N 9/24*** *(2006.01)* ***C12N 15/82*** *(2006.01)*
***C12Q 1/40*** *(2006.01)* ***C12P 7/14*** *(2006.01)*

(86) International application number:
**PCT/EP2015/065752**

(87) International publication number:
**WO 2016/005521 (14.01.2016 Gazette 2016/02)**

(54) **PROCESS FOR PRODUCING ETHANOL FROM STARCH USING A GH5 XYLANASE**

VERFAHREN ZUM HERSTELLEN VON ETHANOL AUS STÄRKE UNTER VERWENDUNG EINER GH5 XYLANASE

PROCÉDÉ DE PRODUCTION DE L'ÉTHANOL À PARTIR DE L'AMIDON À L'AIDE D'UNE XYLANASE GH5

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.07.2014 EP 14176473**
**10.07.2014 EP 14176468**
**20.10.2014 EP 14189543**

(43) Date of publication of application:
**17.05.2017 Bulletin 2017/20**

(73) Proprietor: **Novozymes A/S**
**2880 Bagsvaerd (DK)**

(72) Inventors:
- **PEDERSEN, Sven**
**DK-2880 Bagsvaerd (DK)**
- **EKLOEF, Jens**
**DK-2880 Bagsvaerd (DK)**

(74) Representative: **NZ EPO Representatives**
**Krogshoejvej 36**
**2880 Bagsvaerd (DK)**

(56) References cited:
**WO-A1-2005/059084 WO-A1-2005/121305**
**WO-A1-2009/117689 WO-A1-2012/084225**

- **M. A. S. CORREIA ET AL: "Structure and Function of an Arabinoxylan-specific Xylanase", JOURNAL OF BIOLOGICAL CHEMISTRY, vol. 286, no. 25, 24 June 2011 (2011-06-24), pages 22510-22520, XP055087490, ISSN: 0021-9258, DOI: 10.1074/jbc.M110.217315**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> Novozymes A/S

<120> Process for producing ethanol from starch using a GH5 xylanase

<130> 12986-WO-PCT

<160> 8

<170> PatentIn version 3.5

<210> 1
<211> 1722
<212> DNA
<213> Artificial

<220>
<223> Codon optimized gene encoding a GH5_35 xylanase from Paenibacillus illinoisensis

<400> 1

atgaagaaac cgttggggaa aattgtcgca agcaccgcac tactcatttc tgttgctttt 60
agttcatcga tcgcatcggc tcatcagcac caacaccagc atcctaggtg ggttggcatg 120
ccgatgggca aactgcatgt ttcaggcaaa aatctggtca atagcaataa tcaaccggtt 180
ctgctgaatg gctggcatca accgtcaggc gcatattgga catatcaatc atcaaactat 240
tatctgaatc tgcatggaaa taatcgccat gcggcaacac tggcatatct gaaagatatc 300
acagatacat ttgcagacac gtcaccgaaa tatggctcaa atcatggctg gaatatgaat 360
caggtccgcc tgtttattga tcgcgaagat atgggagatg ttgcagcagg cacatataac 420
tttgcaggcg ttcaaacagt tacacagaat gtcattattc cgtatatcca gtatgcgaaa 480
acgaaaggcg tttatgttgt tctgggcctg gattttacac tgaaagatga tcaagcaaca 540
acagcgagca atctgcagaa atttaaccaa atttggggct atctggcatc aagaccggaa 600
atcaaatcag cagataacgt ccattttgaa ctgattaacg aaccggttaa aagctatgca 660
aatggccatt ggggaggcta taatggcgaa aatgattttg tcgatcattg gaatgatctg 720
cgcaattttc agaacagcat tatttcaaca attcgctcac aaggcgcaga taatgttatt 780
tgggcagcag gcctgggcta taatcagttt tattcactga cagcatcaca tccgctgaca 840
gatccgctga ataactatgg ctatgcagtt cattggtatc cgggatatgg cgcatatgat 900
aactttagca ttctgcagga tcagtggaac acaaatgtta aagcagcagc agataaatat 960
ccgattaaca tcacggaagt cacatggttt aaaaacaaac ctggcgattc agcatactgg 1020
aatcttttta atggcagcaa tgaaggcttt ggcacaaaca caaaaacgat ctttaatgca 1080
gcaggcaatg ttagcattgc agcacatatg aatggcttta ttctggaacc gggacaaaga 1140
tcatcatttg cagatccgac agcaggcctg aaatgggatg gcgacgcatc aagatcagca 1200
atgggcagat ttctgtttaa ttggtatcat gaaagagcgc aatcatatcc tggcggaaat 1260

ggcggaggac cgacaacagg cctggtttca ggcgcaacat acaaaattgt tgcacgccat 1320
agcaacaaag tcattgatgt ccctggcgga caaaatcaaa acaatctgca actgcaacag 1380
tggtcagatc tgggaggcaa tccgcaaaaa tgggttctga catcaattgg cggaggctca 1440
tatacactga catcagttaa ttcaccggac aaagtgattg atattcgcaa tggcacactg 1500
acaaatggcg aagcagttca actgatgtca aatctgaata caacagcgca gcattttaaa 1560
gtcaatgatc tgggcaatgg ctattggagc attattaacg tcaacagcaa caaagcgatc 1620
gaagttgaaa atgcatcaac atcagatggc gcaaaactgc agcaaaatac atatacaggc 1680
gcaacaaatc agcagtggaa atttatcgca gtcagcaatt aa 1722

<210> 2
<211> 573

<212> PRT
<213> Artificial

<220>
<223> GH5_35 xylanase from Paenibacillus illinoisensis including a signal peptide from Bacillus clausii and a His-tag

<400> 2

```
Met Lys Lys Pro Leu Gly Lys Ile Val Ala Ser Thr Ala Leu Leu Ile
1               5                   10                  15

Ser Val Ala Phe Ser Ser Ser Ile Ala Ser Ala His Gln His Gln His
            20                  25                  30

Gln His Pro Arg Trp Val Gly Met Pro Met Gly Lys Leu His Val Ser
        35                  40                  45

Gly Lys Asn Leu Val Asn Ser Asn Asn Gln Pro Val Leu Leu Asn Gly
    50                  55                  60

Trp His Gln Pro Ser Gly Ala Tyr Trp Thr Tyr Gln Ser Ser Asn Tyr
65                  70                  75                  80

Tyr Leu Asn Leu His Gly Asn Asn Arg His Ala Ala Thr Leu Ala Tyr
                85                  90                  95

Leu Lys Asp Ile Thr Asp Thr Phe Ala Asp Thr Ser Pro Lys Tyr Gly
            100                 105                 110

Ser Asn His Gly Trp Asn Met Asn Gln Val Arg Leu Phe Ile Asp Arg
        115                 120                 125

Glu Asp Met Gly Asp Val Ala Ala Gly Thr Tyr Asn Phe Ala Gly Val
    130                 135                 140
```

Gln Thr Val Thr Gln Asn Val Ile Ile Pro Tyr Ile Gln Tyr Ala Lys
145 150 155 160

Thr Lys Gly Val Tyr Val Val Leu Gly Leu Asp Phe Thr Leu Lys Asp
165 170 175

Asp Gln Ala Thr Thr Ala Ser Asn Leu Gln Lys Phe Asn Gln Ile Trp
180 185 190

Gly Tyr Leu Ala Ser Arg Pro Glu Ile Lys Ser Ala Asp Asn Val His
195 200 205

Phe Glu Leu Ile Asn Glu Pro Val Lys Ser Tyr Ala Asn Gly His Trp
210 215 220

Gly Gly Tyr Asn Gly Glu Asn Asp Phe Val Asp His Trp Asn Asp Leu
225 230 235 240

Arg Asn Phe Gln Asn Ser Ile Ile Ser Thr Ile Arg Ser Gln Gly Ala
245 250 255

Asp Asn Val Ile Trp Ala Ala Gly Leu Gly Tyr Asn Gln Phe Tyr Ser
260 265 270

Leu Thr Ala Ser His Pro Leu Thr Asp Pro Leu Asn Asn Tyr Gly Tyr
275 280 285

Ala Val His Trp Tyr Pro Gly Tyr Gly Ala Tyr Asp Asn Phe Ser Ile
290 295 300

Leu Gln Asp Gln Trp Asn Thr Asn Val Lys Ala Ala Ala Asp Lys Tyr
305 310 315 320

Pro Ile Asn Ile Thr Glu Val Thr Trp Phe Lys Asn Lys Pro Gly Asp
325 330 335

Ser Ala Tyr Trp Asn Leu Phe Asn Gly Ser Asn Glu Gly Phe Gly Thr
340 345 350

Asn Thr Lys Thr Ile Phe Asn Ala Ala Gly Asn Val Ser Ile Ala Ala
355 360 365

His Met Asn Gly Phe Ile Leu Glu Pro Gly Gln Arg Ser Ser Phe Ala
370 375 380

Asp Pro Thr Ala Gly Leu Lys Trp Asp Gly Asp Ala Ser Arg Ser Ala

```
385                 390                 395                 400

Met Gly Arg Phe Leu Phe Asn Trp Tyr His Glu Arg Ala Gln Ser Tyr
                405                 410                 415

Pro Gly Gly Asn Gly Gly Gly Pro Thr Thr Gly Leu Val Ser Gly Ala
            420                 425                 430

Thr Tyr Lys Ile Val Ala Arg His Ser Asn Lys Val Ile Asp Val Pro
        435                 440                 445

Gly Gly Gln Asn Gln Asn Asn Leu Gln Leu Gln Gln Trp Ser Asp Leu
    450                 455                 460

Gly Gly Asn Pro Gln Lys Trp Val Leu Thr Ser Ile Gly Gly Gly Ser
465                 470                 475                 480

Tyr Thr Leu Thr Ser Val Asn Ser Pro Asp Lys Val Ile Asp Ile Arg
                485                 490                 495

Asn Gly Thr Leu Thr Asn Gly Glu Ala Val Gln Leu Met Ser Asn Leu
            500                 505                 510

Asn Thr Thr Ala Gln His Phe Lys Val Asn Asp Leu Gly Asn Gly Tyr
        515                 520                 525

Trp Ser Ile Ile Asn Val Asn Ser Asn Lys Ala Ile Glu Val Glu Asn
    530                 535                 540

Ala Ser Thr Ser Asp Gly Ala Lys Leu Gln Gln Asn Thr Tyr Thr Gly
545                 550                 555                 560

Ala Thr Asn Gln Gln Trp Lys Phe Ile Ala Val Ser Asn
                565                 570
```

<210> 3
<211> 1749
<212> DNA
<213> Artificial

<220>
<223> Codon optimized gene encoding a GH5_35 xylanase from Paenibacillus sp 18054

<400> 3

atgaagaaac cgttggggaa aattgtcgca agcaccgcac tactcatttc tgttgctttt 60

agttcatcga tagcatcagc acatcatcat caccatcatc ctaggttgac ggttccgccc 120

ggcgccccgg ccgaggcttg gtctggcatg cctacgccca agcttcatgt cagcggcaac 180

caactggtaa atgcaaacgg acagcctgtc ctgctaagcg ggtggcatca gccttccggg 240

tcctactgga cgtatcagag cagcagttac tacctggacc gtaacggcgg aaaccggcat 300

gccgccaacc tggcgtatct caaggatatc acagacacct ttacggacac ctcgcccaaa 360

tacgggaaca accacggctg gtatatgaat caggttcggc tattcattga tcgcgaggat 420

atgggggatg tcgccgaagg cacttacaac tttgctggat tgcaggcggt tacgcaaaat 480

gtgattattc cgtacattaa ttatgcgaga acaaaagggc tttatgtgac gctcggactg 540

gattttacgc tcaaagacaa tcaggcgacc acacaggcca atttggacaa gttcaatcag 600

atttggagtt atctagcatc cagaccggaa ataagaagcg ctgacaacgt catgtttgaa 660

attattaacg agccggtatt gtcgtatgcg gacggcagat ggggcggtca tccgtccgac 720

cctcatttta tagcattctg gaacgatttg cgcagttttc aaaactctat catctcctcc 780

attcgcgcac aaggagcgga taatgtgatc tgggcggccg ggctcggctg ggatcagtac 840

taccagttgt gtgcgtcgca tcctctgacg gacccgctca ataatgtagg ttatgcggtt 900

cactggtatc cgggatatgg agcaggggat aactattcgg tgcttcagca acaatgggat 960

acgaatatta agccatgcgc cgacaactat ccgatcaata taacggaaac gacctggttc 1020

aagcgactgc ctggcgattc ggattactgg aatttgttca atggctcgag cgagggcttc 1080

ggcaaaaata cgaaagcaat ctttactgcg gcgggcaatg ccagcattgc cgtccatatg 1140

aacggctttt tattggcgcc gggagcaaga agctcatttg ccgatccgac tgccggtctg 1200

ttatacgacg gaaatacagc tcgagacggc atggcccgct ttatattcga gtggtattac 1260

gaaagagcgc agttcttgcc gtggaatggc atttggaacg gactgttcac aggatcgacc 1320

tacaagttcg tgaatcgggc aaccggcaaa aatatggatg tgccgggcgg acaaaacaat 1380

aataatctgc aattgaacca atggacggat aatggagcaa cggcgcagcg ctgggtcgta 1440

gacgatatgg gcactttcaa caatatatat cgcatgaaga gcgtcagctc atcggacggc 1500

aaggtaatgg atgtccgcaa cggcaccaaa aacaatggag aagccattca gcttatgcag 1560

gacttctcga acacggcgca gcgtttccgg attattcgac ttagcaacgg ctattggagc 1620

attattaatg tcaacagcaa caaagcggtt gaggtcgccg gcggcgcttc tcatgacgga 1680

gcgctattgc aacagaacat gtatcgcgga gatcatcatc aacagtggca gctggttcag 1740

attcaataa 1749

<210> 4

<211> 582
<212> PRT
<213> Artificial

<220>
<223> GH5_35 xylanase from Paenibacillus sp. including a signal peptide from Bacillus clausii and a His-tag

<400> 4

Met Lys Lys Pro Leu Gly Lys Ile Val Ala Ser Thr Ala Leu Leu Ile
1               5                   10                  15

Ser Val Ala Phe Ser Ser Ser Ile Ala Ser Ala His His His His His
            20                  25                  30

His Pro Arg Leu Thr Val Pro Pro Gly Ala Pro Ala Glu Ala Trp Ser
        35                  40                  45

Gly Met Pro Thr Pro Lys Leu His Val Ser Gly Asn Gln Leu Val Asn
    50                  55                  60

Ala Asn Gly Gln Pro Val Leu Leu Ser Gly Trp His Gln Pro Ser Gly
65                  70                  75                  80

Ser Tyr Trp Thr Tyr Gln Ser Ser Ser Tyr Tyr Leu Asp Arg Asn Gly
                85                  90                  95

Gly Asn Arg His Ala Ala Asn Leu Ala Tyr Leu Lys Asp Ile Thr Asp
            100                 105                 110

Thr Phe Thr Asp Thr Ser Pro Lys Tyr Gly Asn Asn His Gly Trp Tyr
        115                 120                 125

Met Asn Gln Val Arg Leu Phe Ile Asp Arg Glu Asp Met Gly Asp Val
    130                 135                 140

Ala Glu Gly Thr Tyr Asn Phe Ala Gly Leu Gln Ala Val Thr Gln Asn
145                 150                 155                 160

Val Ile Ile Pro Tyr Ile Asn Tyr Ala Arg Thr Lys Gly Leu Tyr Val
                165                 170                 175

Thr Leu Gly Leu Asp Phe Thr Leu Lys Asp Asn Gln Ala Thr Thr Gln
            180                 185                 190

Ala Asn Leu Asp Lys Phe Asn Gln Ile Trp Ser Tyr Leu Ala Ser Arg
        195                 200                 205

Pro Glu Ile Arg Ser Ala Asp Asn Val Met Phe Glu Ile Ile Asn Glu
    210                 215                 220

Pro Val Leu Ser Tyr Ala Asp Gly Arg Trp Gly Gly His Pro Ser Asp
225                 230                 235                 240

```
Pro His Phe Ile Ala Phe Trp Asn Asp Leu Arg Ser Phe Gln Asn Ser
                245                 250                 255

Ile Ile Ser Ser Ile Arg Ala Gln Gly Ala Asp Asn Val Ile Trp Ala
            260                 265                 270

Ala Gly Leu Gly Trp Asp Gln Tyr Tyr Gln Leu Cys Ala Ser His Pro
        275                 280                 285

Leu Thr Asp Pro Leu Asn Asn Val Gly Tyr Ala Val His Trp Tyr Pro
    290                 295                 300

Gly Tyr Gly Ala Gly Asp Asn Tyr Ser Val Leu Gln Gln Gln Trp Asp
305                 310                 315                 320

Thr Asn Ile Lys Pro Cys Ala Asp Asn Tyr Pro Ile Asn Ile Thr Glu
                325                 330                 335

Thr Thr Trp Phe Lys Arg Leu Pro Gly Asp Ser Asp Tyr Trp Asn Leu
            340                 345                 350

Phe Asn Gly Ser Ser Glu Gly Phe Gly Lys Asn Thr Lys Ala Ile Phe
        355                 360                 365

Thr Ala Ala Gly Asn Ala Ser Ile Ala Val His Met Asn Gly Phe Leu
    370                 375                 380

Leu Ala Pro Gly Ala Arg Ser Ser Phe Ala Asp Pro Thr Ala Gly Leu
385                 390                 395                 400

Leu Tyr Asp Gly Asn Thr Ala Arg Asp Gly Met Ala Arg Phe Ile Phe
                405                 410                 415

Glu Trp Tyr Tyr Glu Arg Ala Gln Phe Leu Pro Trp Asn Gly Ile Trp
            420                 425                 430

Asn Gly Leu Phe Thr Gly Ser Thr Tyr Lys Phe Val Asn Arg Ala Thr
        435                 440                 445

Gly Lys Asn Met Asp Val Pro Gly Gly Gln Asn Asn Asn Asn Leu Gln
    450                 455                 460

Leu Asn Gln Trp Thr Asp Asn Gly Ala Thr Ala Gln Arg Trp Val Val
465                 470                 475                 480

Asp Asp Met Gly Thr Phe Asn Asn Ile Tyr Arg Met Lys Ser Val Ser
                485                 490                 495
```

```
Ser Ser Asp Gly Lys Val Met Asp Val Arg Asn Gly Thr Lys Asn Asn
            500                 505                 510

Gly Glu Ala Ile Gln Leu Met Gln Asp Phe Ser Asn Thr Ala Gln Arg
        515                 520                 525

Phe Arg Ile Ile Arg Leu Ser Asn Gly Tyr Trp Ser Ile Ile Asn Val
    530                 535                 540

Asn Ser Asn Lys Ala Val Glu Val Ala Gly Gly Ala Ser His Asp Gly
545                 550                 555                 560

Ala Leu Leu Gln Gln Asn Met Tyr Arg Gly Asp His His Gln Gln Trp
                565                 570                 575

Gln Leu Val Gln Ile Gln
            580
```

<210> 5
<211> 1902
<212> DNA
<213> Artificial

<220>
<223> Codon optimized gene encoding GH5_21 xylanase from unknown organism

<400> 5

```
atgaagaaac cgttggggaa aattgtcgca agcaccgcac tactcatttc tgttgctttt      60
agttcatcga tagcatcagc acatcatcat caccatcatc ctaggtggcg tggcatgaga     120
atgccggaac tgtttatcaa aggcagatat ctgatggcga aagatatgaa tggcaacgat     180
agcattgtta atctgcatgg ctttggccaa acatatagcg cgtattttaa cggctatgcg     240
tggtgcaaaa atccggatgg ctcagttaat tggggcaaaa caaaagatgc agcagcatgc     300
gttaaatgga ataaagaaca aattggcctg atgctggatc atggctggaa agttaattgg     360
ctgagactgc atatggatcc ggcatggtca aataatgaaa caaaagtcaa tcaatggcag     420
agccaacatc cgggaacata ttattcagaa aatctgatcg tcgcgtttga tatgaacctg     480
tttaaaaaat atctggatga aatctttatt ccgatggcgg aatatgcgat tgaaaacggc     540
atttatgttg ttatgcgtcc gcctggcgtt tgtccgcaaa aactgacagt tggagatgaa     600
tatcagcagt acctgattaa agtctggaca tatgtttgca gccatgaaaa actgaaaaac     660
aatccgtata ttatgtttga actggcgaac gaaccgatcg atatgaatga tggcaatggc     720
aattatacgt catggtcaga tggctcacag aaaaactgca cgaaattttt tcagaaaatt     780
gtcgacgaaa ttagagcagt cggctgcaat aacattctgt gggttccggg actggcatat     840
```

```
caacaaaatt atcaaggcta tgtcaaatac ccgattgtcg gcgaaaatat tggctttgca      900
gttcattgct atccgggatg gtatggctca gattcagaag ttgcatcagc agaacaacaa      960
attgtcacaa acggcaatac gtatgcggat tttcaatcag gctggtcagc aagcattgat     1020
ggcgtttcaa aacttagacc gattatcgtc acagaaatgg attgggcacc gaaaaaatac     1080
aattcatcat ggggcaaagc aacgacaggc aaactgggag gcgttggctt tggcaataac     1140
tttaaataca tcatggacaa aacaggcaac gttagctgga tgctgtttac agatgcagat     1200
aaactggcga aatatgatga ttcaaaagca gatggcagca cgtttctgac agatccggaa     1260
gcatgcccta gaccggttta tagatggtat aaagaatatg cagaaccggg atggaaattt     1320
gttgaaacac tggcagatga attttacatg tttccgggaa caaacagcat ttttagcccg     1380
aacatttggg aaaaaggcac actgacaaaa aatgatgatg gctcaagaac actggtcaca     1440
ggccaatatg gctttggagg ctggaaattt ggcggaggcc tggatatgtc aggctataaa     1500
tacctggttc tgaacctgac aaaagcaccg gcatcaaatc aatggtcact gagactgttt     1560
gatgtcgata actattggac agacccgtat atgaaagatg tcaaatcaag cacaagagtc     1620
gttgtcgatc tgcagaatat gaaaaatagc aaaggcgtta aagtcgaccc gagccatatc     1680
tatattctgg gcctgtggtc aacaggcgga acaccgatta caattaaaga tatctatctg     1740
acaaataata gcgattattc accggaatca acaggcattt cagaaacact tgcagaaaaa     1800
agactggaca caccgattta taacctgtca ggccaaagag ttacagaacc gagaaatggc     1860
catgtctata ttcgcaacgg caaaaaattc atttacaaat aa                        1902
```

<210> 6
<211> 633
<212> PRT
<213> Artificial

<220>
<223> GH5_21 xylanase including a signal peptide from Bacillus clausii and a His tag

<400> 6

```
Met Lys Lys Pro Leu Gly Lys Ile Val Ala Ser Thr Ala Leu Leu Ile
1               5                   10                  15

Ser Val Ala Phe Ser Ser Ser Ile Ala Ser Ala His His His His His
            20                  25                  30

His Pro Arg Trp Arg Gly Met Arg Met Pro Glu Leu Phe Ile Lys Gly
        35                  40                  45

Arg Tyr Leu Met Ala Lys Asp Met Asn Gly Asn Asp Ser Ile Val Asn
    50                  55                  60
```

```
Leu His Gly Phe Gly Gln Thr Tyr Ser Ala Tyr Phe Asn Gly Tyr Ala
65                  70                  75                  80

Trp Cys Lys Asn Pro Asp Gly Ser Val Asn Trp Gly Lys Thr Lys Asp
                85                  90                  95

Ala Ala Ala Cys Val Lys Trp Asn Lys Glu Gln Ile Gly Leu Met Leu
            100                 105                 110

Asp His Gly Trp Lys Val Asn Trp Leu Arg Leu His Met Asp Pro Ala
        115                 120                 125

Trp Ser Asn Asn Glu Thr Lys Val Asn Gln Trp Gln Ser Gln His Pro
    130                 135                 140

Gly Thr Tyr Tyr Ser Glu Asn Leu Ile Val Ala Phe Asp Met Asn Leu
145                 150                 155                 160

Phe Lys Lys Tyr Leu Asp Glu Ile Phe Ile Pro Met Ala Glu Tyr Ala
                165                 170                 175

Ile Glu Asn Gly Ile Tyr Val Val Met Arg Pro Pro Gly Val Cys Pro
            180                 185                 190

Gln Lys Leu Thr Val Gly Asp Glu Tyr Gln Gln Tyr Leu Ile Lys Val
        195                 200                 205

Trp Thr Tyr Val Cys Ser His Glu Lys Leu Lys Asn Asn Pro Tyr Ile
    210                 215                 220

Met Phe Glu Leu Ala Asn Glu Pro Ile Asp Met Asn Asp Gly Asn Gly
225                 230                 235                 240

Asn Tyr Thr Ser Trp Ser Asp Gly Ser Gln Lys Asn Cys Thr Lys Phe
                245                 250                 255

Phe Gln Lys Ile Val Asp Glu Ile Arg Ala Val Gly Cys Asn Asn Ile
            260                 265                 270

Leu Trp Val Pro Gly Leu Ala Tyr Gln Gln Asn Tyr Gln Gly Tyr Val
        275                 280                 285

Lys Tyr Pro Ile Val Gly Glu Asn Ile Gly Phe Ala Val His Cys Tyr
    290                 295                 300

Pro Gly Trp Tyr Gly Ser Asp Ser Glu Val Ala Ser Ala Glu Gln Gln
305                 310                 315                 320
```

```
Ile Val Thr Asn Gly Asn Thr Tyr Ala Asp Phe Gln Ser Gly Trp Ser
                325                 330                 335

Ala Ser Ile Asp Gly Val Ser Lys Leu Arg Pro Ile Ile Val Thr Glu
            340                 345                 350

Met Asp Trp Ala Pro Lys Lys Tyr Asn Ser Ser Trp Gly Lys Ala Thr
        355                 360                 365

Thr Gly Lys Leu Gly Gly Val Gly Phe Gly Asn Asn Phe Lys Tyr Ile
    370                 375                 380

Met Asp Lys Thr Gly Asn Val Ser Trp Met Leu Phe Thr Asp Ala Asp
385                 390                 395                 400

Lys Leu Ala Lys Tyr Asp Asp Ser Lys Ala Asp Gly Ser Thr Phe Leu
                405                 410                 415

Thr Asp Pro Glu Ala Cys Pro Arg Pro Val Tyr Arg Trp Tyr Lys Glu
            420                 425                 430

Tyr Ala Glu Pro Gly Trp Lys Phe Val Glu Thr Leu Ala Asp Glu Phe
        435                 440                 445

Tyr Met Phe Pro Gly Thr Asn Ser Ile Phe Ser Pro Asn Ile Trp Glu
    450                 455                 460

Lys Gly Thr Leu Thr Lys Asn Asp Asp Gly Ser Arg Thr Leu Val Thr
465                 470                 475                 480

Gly Gln Tyr Gly Phe Gly Gly Trp Lys Phe Gly Gly Gly Leu Asp Met
                485                 490                 495

Ser Gly Tyr Lys Tyr Leu Val Leu Asn Leu Thr Lys Ala Pro Ala Ser
            500                 505                 510

Asn Gln Trp Ser Leu Arg Leu Phe Asp Val Asp Asn Tyr Trp Thr Asp
        515                 520                 525

Pro Tyr Met Lys Asp Val Lys Ser Ser Thr Arg Val Val Val Asp Leu
    530                 535                 540

Gln Asn Met Lys Asn Ser Lys Gly Val Lys Val Asp Pro Ser His Ile
545                 550                 555                 560

Tyr Ile Leu Gly Leu Trp Ser Thr Gly Gly Thr Pro Ile Thr Ile Lys
```

565 570 575

Asp Ile Tyr Leu Thr Asn Asn Ser Asp Tyr Ser Pro Glu Ser Thr Gly
580 585 590

Ile Ser Glu Thr Leu Ala Glu Lys Arg Leu Asp Thr Pro Ile Tyr Asn
595 600 605

Leu Ser Gly Gln Arg Val Thr Glu Pro Arg Asn Gly His Val Tyr Ile
610 615 620

Arg Asn Gly Lys Lys Phe Ile Tyr Lys
625 630

<210> 7
<211> 620
<212> PRT
<213> Acetivibrio cellulyticus

<400> 7

```
Ala Asp Pro Gln Arg Gly Arg Pro Tyr Leu Asn Ser Ala Arg Thr Thr
1               5                   10                  15

Phe Val Gly Asp Asn Gly Gln Pro Leu Arg Gly Pro Tyr Ile Ser Thr
            20                  25                  30

Glu Trp Thr Ser Ala Ala Pro Tyr Asp Gln Ile Ala Arg Ile Lys Asn
        35                  40                  45

Leu Gly Phe Asn Ala Val His His Tyr Ala Glu Cys Phe Asp Ile Asn
    50                  55                  60

Tyr Pro Asn Ala Gly Ser Lys Ser Pro Gly Tyr Ala Ala Thr Glu Ile
65                  70                  75                  80

Asp Lys Val Val Glu Arg Thr Arg Glu Leu Gly Leu Tyr Leu Val Met
                85                  90                  95

Thr Ile Gly Asn Gly Ala Asn Asn Gly Asn His Asn Thr Arg Tyr Ala
            100                 105                 110

Lys Asp Phe Trp Ser Phe Tyr Ser Ser Arg Tyr Ala Asn Glu Thr His
        115                 120                 125

Val Leu Tyr Glu Ile His Asn Glu Pro Val Ala Trp Gly Pro Pro Tyr
    130                 135                 140

Ser Ser Thr Thr Ala Thr Pro Thr Gly Ala Val Glu Met Asn Val Asp
```

```
145                 150                 155                 160

Val Tyr Lys Thr Ile Arg Ala Asn Ala Pro Lys Thr Pro Val Leu Ile
                165                 170                 175

Phe Ser Tyr Ser Val Phe Gly Gly Thr Gly Gly Thr Thr Glu Ala Leu
            180                 185                 190

Lys Asp Ile Gln Ala Phe Asn Ser Ala Val Phe Gly Lys Gln Asp Ala
        195                 200                 205

Val Trp Thr Asn Glu Ala Val Ala Phe His Gly Tyr Ala Gly Trp Glu
    210                 215                 220

Ala Thr Ser Thr Ala Val Asp Gly Leu Leu Lys Ala Gly Tyr Pro Cys
225                 230                 235                 240

Phe Met Thr Glu Tyr Ala Gly Gly Ala Trp Gly Ser Gly Thr Gly Gly
                245                 250                 255

Phe Asp Ile Gln Leu Thr Ser Glu Leu Glu Arg Met Gly Val Ser Trp
            260                 265                 270

Leu Thr Phe Gln Tyr Ile Pro Pro Ser Gly Val Ser Asp Asp Val Thr
        275                 280                 285

Lys Pro Glu Tyr Phe Ser Ala Leu Val Glu Asn Ala Gly Leu Ser Trp
    290                 295                 300

Lys Pro Asp Tyr Gly Asn Trp Pro Ala Ala Arg Gly Val His Gly Asn
305                 310                 315                 320

Gly Gly Leu Pro Arg Lys Thr Ser Thr Trp Val Asn Asn Phe Leu Thr
                325                 330                 335

Gly Thr Thr Arg Ile Glu Ala Glu Asp Phe Asp Trp Gly Gly Asn Asp
            340                 345                 350

Val Ser Phe Tyr Asp Lys Asp Ser Glu Asn Lys Gly Ala Gln Tyr Arg
        355                 360                 365

Leu Asp Glu Ala Val Asp Ile Glu Thr Thr Lys Asp Ala Asp Gly Gly
    370                 375                 380

Tyr Asn Val Gly Trp Ile Glu Asp Gly Glu Trp Leu Glu Tyr Thr Ile
385                 390                 395                 400
```

Trp Val Gln His Pro Gly Tyr Tyr Asn Leu Ala Leu Arg Val Ala Asn
405 410 415

Asn Ser Gly Gly Ser Val Gln Val Asn Phe Gly Asn Gln Asp Lys Thr
420 425 430

Gly Thr Trp Val Leu Pro Val Thr Gly Gly Val Gln Thr Trp Lys Thr
435 440 445

Asp Thr Arg Gln Val Phe Leu Gly Ser Gly Arg Gln Lys Leu Arg Ile
450 455 460

Asn Ala Leu Ser Gly Gly Phe Asn Leu Asn Trp Ile Glu Leu Ser Pro
465 470 475 480

Val Ser Thr Gly Pro Ile Ala Asp Gly Thr Tyr Lys Phe Leu Asn Arg
485 490 495

Ala Asn Thr Met Thr Leu Gln Glu Val Thr Ser Asn Asn Ser Ile Val
500 505 510

Thr Ser Thr Tyr Lys Gly Thr Ala Asp Gln His Trp Lys Ile Gln His
515 520 525

Ile Gly Gly Gly Gln Tyr Arg Ile Ser Ser Ala Gly Arg Gly Trp Asn
530 535 540

Trp Asn Trp Trp Met Gly Phe Gly Thr Val Gly Trp Trp Gly Thr Gly
545 550 555 560

Ser Gly Thr Cys Phe Ile Ile Arg Pro Thr Gly Asp Gly Tyr Tyr Arg
565 570 575

Phe Val Leu Val Asn Asp Gly Thr Asn Leu Glu Ile Ser Asn Asn Asp
580 585 590

Ser Ser Lys Ile Glu Gly Lys Ala Tyr His Glu Gly Ala Asn Gln Gln
595 600 605

Trp Ala Ile Gln Leu Pro Ser Ala Pro Val Phe Pro
610 615 620

<210> 8
<211> 865
<212> PRT
<213> Unknown

<220>

<223> Commercial GH5_34 xylanase from NZYtech

<400> 8

```
Ala Ile Ala Leu Ser Ile Leu Ser Phe Ile Pro Asn Arg Ala Tyr Ala
1               5                   10                  15

Ser Pro Gln Arg Gly Arg Pro Arg Leu Asn Ala Ala Arg Thr Thr Phe
            20                  25                  30

Val Gly Asp Asn Gly Gln Pro Leu Arg Gly Pro Tyr Thr Ser Thr Glu
        35                  40                  45

Trp Thr Ala Ala Ala Pro Tyr Asp Gln Ile Ala Arg Val Lys Glu Leu
    50                  55                  60

Gly Phe Asn Ala Val His Leu Tyr Ala Glu Cys Phe Asp Pro Arg Tyr
65                  70                  75                  80

Pro Ala Pro Gly Ser Lys Ala Pro Gly Tyr Ala Val Asn Glu Ile Asp
                85                  90                  95

Lys Ile Val Glu Arg Thr Arg Glu Leu Gly Leu Tyr Leu Val Ile Thr
            100                 105                 110

Ile Gly Asn Gly Ala Asn Asn Gly Asn His Asn Ala Gln Trp Ala Arg
        115                 120                 125

Asp Phe Trp Lys Phe Tyr Ala Pro Arg Tyr Ala Lys Glu Thr His Val
    130                 135                 140

Leu Tyr Glu Ile His Asn Glu Pro Val Ala Trp Gly Pro Pro Tyr Ser
145                 150                 155                 160

Ser Ser Thr Ala Asn Pro Pro Gly Ala Val Asp Met Glu Ile Asp Val
                165                 170                 175

Tyr Arg Ile Ile Arg Thr Tyr Ala Pro Glu Thr Pro Val Leu Leu Phe
            180                 185                 190

Ser Tyr Ala Val Phe Gly Gly Lys Gly Gly Ala Ala Glu Ala Leu Lys
        195                 200                 205

Asp Ile Arg Ala Phe Asn Lys Ala Val Phe Gly Asn Glu Asn Ala Val
    210                 215                 220

Trp Thr Asn Glu Ala Val Ala Phe His Gly Tyr Ala Gly Trp Gln Glu
225                 230                 235                 240
```

```
Thr Thr Ile Ala Val Glu Glu Leu Leu Lys Ala Gly Tyr Pro Cys Phe
                245                 250                 255

Met Thr Glu Tyr Ala Gly Gly Ala Trp Gly Ser Gly Met Gly Gly Leu
            260                 265                 270

Asp Val Glu Leu Thr Tyr Glu Leu Glu Arg Leu Gly Val Ser Trp Leu
        275                 280                 285

Thr Phe Gln Tyr Ile Pro Pro Thr Gly Val Ser Asp Asp Val Thr Lys
    290                 295                 300

Pro Glu Tyr Phe Ser Ala Leu Val Glu Asn Ser Gly Leu Ser Trp Thr
305                 310                 315                 320

Pro Asp Tyr Gly Asn Trp Pro Ala Ala Arg Gly Val Tyr Gly Asn Gly
                325                 330                 335

Gly Leu Ala Arg Glu Thr Ala Thr Trp Ile Asn Asn Phe Leu Thr Gly
            340                 345                 350

Thr Thr Arg Ile Glu Ala Glu Asp Phe Asp Trp Gly Gly Asn Gly Val
        355                 360                 365

Ser Tyr Tyr Asp Thr Asp Ser Val Asn Val Gly Gly Gln Tyr Arg Pro
    370                 375                 380

Asp Glu Gly Val Asp Ile Glu Lys Thr Ser Asp Thr Gly Gly Gly Tyr
385                 390                 395                 400

Asn Val Gly Trp Ile Ser Glu Gly Glu Trp Leu Glu Tyr Thr Ile Arg
                405                 410                 415

Val Arg Asn Pro Gly Tyr Tyr Asn Leu Ser Leu Arg Val Ala Gly Ile
            420                 425                 430

Ser Gly Ser Arg Val Gln Val Ser Phe Gly Asn Gln Asp Lys Thr Gly
        435                 440                 445

Val Trp Glu Leu Pro Ala Thr Gly Gly Phe Gln Thr Trp Thr Thr Ala
    450                 455                 460

Thr Arg Gln Val Phe Leu Gly Ala Gly Leu Gln Lys Leu Arg Ile Asn
465                 470                 475                 480

Ala Leu Ser Gly Gly Phe Asn Leu Asn Trp Ile Glu Leu Ser Pro Ile
                485                 490                 495
```

```
Ser Thr Gly Thr Ile Pro Asp Gly Thr Tyr Lys Phe Leu Asn Arg Ala
            500                 505                 510

Asn Gly Lys Thr Leu Gln Glu Val Thr Gly Asn Asn Ser Ile Ile Thr
        515                 520                 525

Ala Asp Tyr Lys Gly Ile Thr Glu Gln His Trp Lys Ile Gln His Ile
    530                 535                 540

Gly Gly Gly Gln Tyr Arg Ile Ser Ser Ala Gly Arg Gly Trp Asn Trp
545                 550                 555                 560

Asn Trp Trp Met Gly Phe Gly Thr Val Gly Trp Trp Gly Thr Gly Ser
                565                 570                 575

Ser Thr Cys Phe Ile Ile Ser Pro Thr Gly Asp Gly Tyr Tyr Arg Ile
            580                 585                 590

Val Leu Val Gly Asp Gly Thr Asn Leu Gln Ile Ser Ser Gly Asp Pro
        595                 600                 605

Ser Lys Ile Glu Gly Lys Ala Phe His Gly Gly Ala Asn Gln Gln Trp
    610                 615                 620

Ala Ile Leu Pro Val Ser Ala Pro Ala Phe Pro Thr Gly Leu Ser Ala
625                 630                 635                 640

Val Leu Asp Ser Ser Gly Asn Thr Ala Asn Leu Thr Trp Asn Ala Ala
                645                 650                 655

Pro Gly Ala Asn Ser Tyr Asn Val Lys Arg Ser Thr Lys Ser Gly Gly
            660                 665                 670

Pro Tyr Thr Thr Ile Ala Thr Asn Ile Thr Ser Thr Asn Tyr Thr Asp
        675                 680                 685

Thr Gly Val Ala Thr Gly Thr Lys Tyr Tyr Tyr Val Val Ser Ala Val
    690                 695                 700

Ser Asn Gly Val Glu Thr Leu Asn Ser Ala Glu Ala Ile Leu Gln Tyr
705                 710                 715                 720

Pro Lys Leu Thr Gly Thr Val Ile Gly Thr Gln Gly Ser Trp Asn Asn
                725                 730                 735

Ile Gly Asn Thr Ile His Lys Ala Phe Asp Gly Asp Leu Asn Thr Phe
            740                 745                 750
```

```
Phe Asp Gly Pro Thr Ala Asn Gly Cys Trp Leu Gly Leu Asp Phe Gly
        755                 760                 765

Glu Gly Val Arg Asn Val Ile Thr Gln Ile Lys Phe Cys Pro Arg Ser
    770                 775                 780

Gly Tyr Glu Gln Arg Met Ile Gly Gly Ile Phe Gln Gly Ala Asn Lys
785                 790                 795                 800

Glu Asp Phe Ser Asp Ala Val Thr Leu Phe Thr Ile Thr Ser Leu Pro
                805                 810                 815

Gly Ser Gly Thr Leu Thr Ser Val Asp Val Asp Asn Pro Thr Gly Phe
            820                 825                 830

Arg Tyr Val Arg Tyr Leu Ser Pro Asp Gly Ser Asn Gly Asn Ile Ala
        835                 840                 845

Glu Leu Gln Phe Phe Gly Thr Pro Ala Gly Glu Glu Asn Asp Asp Val
    850                 855                 860

His
865
```

## Description

### Reference to a Sequence Listing

This application contains a Sequence Listing in computer readable form.

### Background of the Invention

### Field of the Invention

The present invention relates to a process for producing a fermentation product from starch-containing material.

### Description of the Related Art

Xylans are hemicelluloses found in all land plants (Popper and Tuohy, Plant Physiology, 2010, 153:373-383). They are especially abundant in secondary cell walls and xylem cells. In grasses, with type II cell walls, glucurono arabinoxylans are the main hemicellulose and are present as soluble or insoluble dietary fiber in many grass based food and feed products.

Plant xylans have a β-1,4-linked xylopyranose backbone that can be substituted at the O2 or O3 position with arabinose, glucuronic acid and acetic acid in a species and tissue specific manner. The starch-rich seeds of the *Panicoideae* with economically important species such as corn and sorghum have special types of highly substituted xylans in their cell walls. Compared to wheat flour, wherein over 60% of the xylosyl units in the arabinoxylan backbone are unsubstituted. In corn kernel xylan, the corresponding percentage of unsubstituted backbone xylosyls is 20-30%, and in sorghum it is 35-40% (Huismann et al. Carbohydrate Polymers, 2000, 42:269-279). Furthermore, in corn and sorghum the xylan side chains can be longer than a single arabinose or glucuronic acid substitution which is common in other xylans. This added side chain complexity is often due to L- and D-galactose and D-xylose sugars bound to the side chain arabinose or glucuronic acid. About every tenth arabinose in corn kernel xylan is also esterified with a ferulic acid and about every fourth xylose carries an acetylation (Agger et al. J. Agric. Food Chem, 2010, 58:6141-6148). All of these factors combined make the highly substituted xylans in corn and sorghum resistant to degradation by traditional xylanases.

The known enzymes responsible for the hydrolysis of the xylan backbone are classified into enzyme families based on sequence similarity (www.cazy.org). The enzymes with mainly endo-xylanase activity have previously been described in Glycoside hydrolase family (GH) 5, 8, 10, 11 and 30. The enzymes within a family share some characteristics such as 3D fold and they usually share the same reaction mechanism. Some GH families have narrow or mono-specific substrate specificities while other families have broad substrate specificities.

Commercially available GH10 and GH11 xylanases are often used to break down the xylose backbone of arabinoxylan. However, such xylanases are sensitive to side chain steric hindrance and whilst they are effective at degrading arabinoxylan from wheat, they are not very effective on the xylan found in the seeds of *Panicoideae* species, such as corn or sorghum. Corn is used around the world in e.g., animal feed and for producing bioethanol, and thus there is a need to discover new polypeptides having xylanase activity that are capable of breaking down the highly branched xylan backbone in the cell wall in order to release more starch which are trapped inside the cell wall.

### Summary of the Invention

According to the invention several xylanases belonging to the GH5 family have been shown to be effective in hydrolyzing highly branched xylan backbones and thus these GH5 xylanases can be advantageously applied in starch to ethanol processes. The present invention therefore relates to a process for producing fermentation products from starch-containing material comprising the steps of:

a) liquefying starch-containing material using an alpha-amylase;
b) saccharifying the liquefied material using a glucoamylase;
c) fermenting using a fermenting organism, wherein saccharification is carried out in the presence of a GH5 xylanase, wherein the GH5 xylanase is selected from subfamily 35.

### Brief Description of the Figures

Figure 1 shows the effect on ethanol yield by adding a GH5 xylanase during saccharification. A liquefied mash was split into three parts and simultaneous saccharification and fermentation performed. Fermentation was followed by weight loss measured twice a day and by HPLC at the end of fermentation.

**Definitions**

**Arabinoxylan-containing material:** The term "Arabinoxylan-containing material" means any material containing arabinoxylan. Arabinoxylan is a hemicellulose found in both the primary and secondary cell walls of plants, including woods and cereal grains, consisting of copolymers of two pentose sugars, arabinose and xylose. The arabinoxylan chain contains a large number of 1,4-linked xylose units. Many xylose units are substituted with 2-, 3- or 2,3-substituted arabinose residues.

Examples of arabinoxylan-containing material are forage, roughage, seeds and grains (either whole or prepared by crushing, milling, etc. from e.g. corn, oats, rye, barley, wheat), trees or hard woods (such as poplar, willow, eucalyptus, palm, maple, birch), bamboo, herbaceous and/or woody energy crops, agricultural food and feed crops, animal feed products, cassava peels, cocoa pods, sugar cane, sugar beet, locust bean pulp, vegetable or fruit pomaces, wood waste, bark, shavings, sawdust, wood pulp, pulping liquor, waste paper, cardboard, construction and demolition wood waste, industrial or municipal waste water solids or sludge, manure, byproduct from brewing and/or fermentation processes, wet distillers grain, dried distillers grain, spent grain, vinasse and bagasse.

Forage as defined herein also includes roughage. Forage is fresh plant material such as hay and silage from forage plants, grass and other forage plants, grass and other forage plants, seaweed, sprouted grains and legumes, or any combination thereof. Examples of forage plants are Alfalfa (Lucerne), birdsfoot trefoil, brassica (e.g. kale, rapeseed (canola), rutabaga (swede), turnip), clover (e.g. alsike clover, red clover, subterranean clover, white clover), grass (e.g. Bermuda grass, brome, false oat grass, fescue, heath grass, meadow grasses, miscanthus, orchard grass, ryegrass, switchgrass, Timothy-grass), corn (maize), hemp, millet, barley, oats, rye, sorghum, soybeans and wheat and vegetables such as beets. Crops suitable for ensilage are the ordinary grasses, clovers, alfalfa, vetches, oats, rye and maize. Forage further includes crop residues from grain production (such as corn stover; straw from wheat, barley, oat, rye and other grains); residues from vegetables like beet tops; residues from oilseed production like stems and leaves form soy beans, rapeseed and other legumes; and fractions from the refining of grains for animal or human consumption or from fuel production or other industries.

Roughage is generally dry plant material with high levels of fiber, such as fiber, bran, husks from seeds and grains and crop residues (such as stover, copra, straw, chaff, sugar beet waste).

Preferred sources of arabinoxylan-containing materials are forage, roughage, seeds and grains, sugar cane, sugar beet and wood pulp.

**cDNA:** The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

**Coding sequence:** The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

**Control sequences:** The term "control sequences" means nucleic acid sequences necessary for expression of a polynucleotide encoding a mature polypeptide of the present invention. Each control sequence may be native (*i.e.*, from the same gene) or foreign (*i.e.*, from a different gene) to the polynucleotide encoding the polypeptide or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

**Expression:** The term "expression" includes any step involved in the production of a polypeptide including, but not limited to, transcription, post-transcriptional modification, translation, post-translational modification, and secretion.

**Expression vector:** The term "expression vector" means a linear or circular DNA molecule that comprises a polynucleotide encoding a polypeptide and is operably linked to control sequences that provide for its expression.

**Highly branched xylan:** The term "highly branched xylan" means that more than 50% of xylosyl units in the arabinoxylan backbone are substituted. This is preferably calculated from linkage analysis as performed in Huismann et al. Carbohydrate Polymers, 2000, 42:269-279.

**Host cell:** The term "host cell" means any cell type that is susceptible to transformation, transfection, transduction, or the like with a nucleic acid construct or expression vector comprising a polynucleotide of the present invention. The term "host cell" encompasses any progeny of a parent cell that is not identical to the parent cell due to mutations that occur during replication.

**Isolated:** The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (*e.g.*, recombinant production in a host cell; multiple copies of a gene encoding the substance; and use of a stronger promoter than the promoter naturally associated with the gene encoding the substance).

**Mature polypeptide:** The term "mature polypeptide" means a polypeptide in its final form following translation and any post-translational modifications, such as N-terminal processing, C-terminal truncation, glycosylation, phosphorylation, etc. In one aspect, the mature polypeptide is amino acids 37 to 573 of SEQ ID NO: 2. Amino acids 1 to 27 of SEQ ID NO: 2 are a signal peptide. Amino acids 28 to 36 of SEQ ID NO: 2 are a his-tag. In another aspect the mature polypeptide is amino acids 36 to 582 of SEQ ID NO: 4. Amino acids 1 to 27 of SEQ ID NO: 4 are a signal peptide. Amino acids 28 to 35 of SEQ ID NO: 4 are a his-tag.

It is known in the art that a host cell may produce a mixture of two of more different mature polypeptides (*i.e.*, with a different C-terminal and/or N-terminal amino acid) expressed by the same polynucleotide. It is also known in the art that different host cells process polypeptides differently, and thus, one host cell expressing a polynucleotide may produce a different mature polypeptide (*e.g.*, having a different C-terminal and/or N-terminal amino acid) as compared to another host cell expressing the same polynucleotide.

**Mature polypeptide coding sequence:** The term "mature polypeptide coding sequence" means a polynucleotide that encodes a mature polypeptide having xylanase activity. In one aspect, the mature polypeptide coding sequence is nucleotides 109 to 1719 of SEQ ID NO: 1. Nucleotides 1 to 81 of SEQ ID NO: 1 encode a signal peptide. Nucleotides 82 to 108 of SEQ ID NO: 1 encode a his-tag. In one aspect, the mature polypeptide coding sequence is nucleotides 106 to 1746 of SEQ ID NO: 3. Nucleotides 1 to 81 of SEQ ID NO: 3 encode a signal peptide. Nucleotides 82 to 105 of SEQ ID NO: 3 encode a his-tag.

**Oligosaccharide composition:** The term "oligosaccharide composition" means olig- and poly saccharides but does not include mono- and disaccharides (DP1 and DP2).

**Nucleic acid construct:** The term "nucleic acid construct" means a nucleic acid molecule, either single- or double-stranded, which is isolated from a naturally occurring gene or is modified to contain segments of nucleic acids in a manner that would not otherwise exist in nature or which is synthetic, which comprises one or more control sequences.

**Operably linked:** The term "operably linked" means a configuration in which a control sequence is placed at an appropriate position relative to the coding sequence of a polynucleotide such that the control sequence directs expression of the coding sequence.

**Percentage solubilised xylose:** The term "percentage solubilised xylose" means the amount of xylose measured in the supernatant after incubation with an enzyme compared to the total amount of xylose present in the substrate before the incubation with the enzyme. The percentage solubilised xylose from defatted destarched maize (DFDSM) may be calculated as described in example 3 herein.

**Sequence Identity:** The relatedness between two amino acid sequences or between two nucleotide sequences is described by the parameter "sequence identity".

For purposes of the present invention, the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 3.0.0 or later. Version 6.1.0 was used. The optional parameters used are gap open penalty of 10, gap extension penalty of 0.5, and the EBLOSUM62 (EMBOSS version of BLOSUM62) substitution matrix. The output of Needle labelled "longest identity" (obtained using the -nobrief option) is used as the percent identity and is calculated as follows:

$$(\text{Identical Residues} \times 100)/(\text{Length of Alignment} - \text{Total Number of Gaps in Alignment})$$

For purposes of the present invention, the degree of sequence identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice et al., 2000, *supra*), preferably version 3.0.0 or later. Version 6.1.0 was used. The optional parameters used are gap open penalty of 10, gap extension penalty of 0.5, and the EDNAFULL (EMBOSS version of NCBI NUC4.4) substitution matrix. The output of Needle labelled "longest identity" (obtained using the -nobrief option) is used as the percent identity and is calculated as follows:

(Identical Deoxyribonucleotides x 100)/(Length of Alignment – Total Number of Gaps in Alignment)

**Xylanase:** The term "xylanase" means a 1,4-beta-D-xylan-xylohydrolase (E.C. 3.2.1.8) that catalyses the endohydrolysis of 1,4-beta-D-xylosidic linkages in xylans. Xylanase activity can be determined with 0.2% AZCL-arabinoxylan as substrate in 0.01% TRITON® X-100 and 200 mM sodium phosphate pH 6 at 37°C. One unit of xylanase activity is defined as 1.0 μmole of azurine produced per minute at 37°C, pH 6 from 0.2% AZCL-arabinoxylan as substrate in 200 mM sodium phosphate pH 6.

**GH5 xylanase:** The xylanases described in this invention belongs to GH5, a GH family with a wide array of substrate specificities. The relationship between sequences within GH5 has been clarified by defining subfamilies of related sequences (Aspeborg et al. BMC Evolutionary Biology, 2012, 12:186). Subdividing a GH family into subfamilies has significantly improved the predictive power for substrate specificity, not only for GH5 (Lombard et al. Nucleic Acids Res, 2014, 42:D490-D495). Two of the subfamilies of GH5, GH5_21 and GH5_34, have been described as xylanases acting on arabinoxylan. Interestingly, these two xylanase subfamilies are not closely related and are the result of convergent evolution. During the course of this work, members of subfamily GH5_35, a subfamily of previously unknown function, has been found to have xylanase activity.

**Detailed Description of the Invention**

The inventors have found that certain xylanases from glycoside hydrolase family 5 (herein referred to as GH5) are surprisingly good at degrading the xylose backbone of sterically hindered arabinoxylan, thereby releasing increased amounts of xylose. In particular GH5 xylanases belonging to subfamiliy GH5_35 have the capacity to access and degrade highly branched xylans (meaning that more than 50% of xylosyl units in the arabinoxylan backbone are substituted) that are resistant to xylanase degradation by xylanases of other GH families. Increased degradation, and thereby increased xylose release, increase accessibility of starch trapped in the xylose structure. Thus the present invention relates to process for producing fermentation products from starch-containing material comprising the steps of:

a) liquefying starch-containing material using an alpha-amylase;
b) saccharifying the liquefied material using a glucoamylase;
c) fermenting using a fermenting organism, wherein saccharification is carried out in the presence of a GH5 xylanase, wherein the GH5 xylanase is selected from subfamily 35.

**GH5 xylanases**

The GH5 xylanases applied in the process of the invention are in one embodiment selected from subfamily 35.

In one specific embodiment the subfamily 35 GH5 xylanase is selected from the xylanase shown as amino acids 37 to 573 of SEQ ID NO: 2, or a GH5 xylanase having at least 75%, at least 80%, at least 85%, at least 90%, at least 95%, at least 96%, at least 97%, at least 98%, at least 99% identity to amino acids 37 to 573 of SEQ ID NO: 2.

In another specific embodiment the subfamily 35 GH5 xylanase is selected from the xylanase shown as amino acids 36 to 582 of SEQ ID NO: 4, or a GH5 xylanase having at least 75%, at least 80%, at least 85%, at least 90%, at least 95%, at least 96%, at least 97%, at least 98%, at least 99% identity to amino acids 36 to 582 of SEQ ID NO: 4.

**Production of Fermentation Products from Starch-Containing Materials**

Processes for producing fermentation products from gelatinized starch-containing material In this aspect the present invention relates to a process for producing a fermentation product, especially ethanol, from starch-containing material, which process includes a liquefaction step and sequentially or simultaneously performed saccharification and fermentation steps.

The invention relates to processes for producing fermentation products from starch-containing material comprising the steps of:

i) liquefying starch-containing material using an alpha-amylase:
ii) saccharifying the liquefied material using a carbohydrate-source generating enzyme;
iii) fermenting using a fermenting organism, wherein saccharification is carried out in the presence of a GH5 xylanase, wherein the GH5 xylanase is selected from subfamily 35.

The fermentation product, such as especially ethanol, may optionally be recovered after fermentation, e.g., by

distillation. Suitable starch-containing starting materials are listed in the section "Starch-Containing Materials"-section below. In an embodiment the starch-containing materials is corn or what. Contemplated enzymes are listed in the "Enzymes"-section below. The liquefaction is carried out in the presence of an alpha-amylase, preferably a bacterial alpha-amylase, especially *Bacillus* alpha-amylase, such as a *Bacillus stearothermophilus* alpha-amylase. The fermenting organism is preferably yeast, preferably a strain of *Saccharomyces,* especially a strain of *Saccharomyces cerevisae.* Suitable fermenting organisms are listed in the "Fermenting Organisms"-section above. In a preferred embodiment steps ii) and iii) are carried out sequentially or simultaneously (*i.e.*, as SSF process).

The aqueous slurry may contain from 10-55 wt.-% dry solids, preferably 25-45 wt.-% dry solids, more preferably 30-40 wt.-% dry solids of starch-containing material. The slurry is heated to above the initial gelatinization temperature. Alpha-amylase, preferably bacterial alpha-amylase, may be added to the slurry. In an embodiment the slurry is also jet-cooked to further gelatinize the slurry before being subjected to an alpha-amylase in liquefaction step i).

The temperature during step (i) is above the initial gelatinization temperature, such as between 80-90°C, such as around 85°C.

In an embodiment liquefaction is carried out as a three-step hot slurry process. The slurry is heated to between 60-95°C, preferably between 80-90°C, and alpha-amylase is added to initiate liquefaction (thinning). Then the slurry is jet-cooked at a temperature between 95-140°C, preferably 105-125°C, for 1-15 minutes, preferably for 3-10 minutes, especially around 5 minutes. The slurry is cooled to 60-95°C, preferably 80-90°C, and more alpha-amylase is added to finalize hydrolysis (secondary liquefaction). The liquefaction process is usually carried out at pH 4.5-6.5, in particular at a pH between 5 and 6. Milled and liquefied starch is known as "mash".

The saccharification in step ii) may be carried out using conditions well known in the art. For instance, a full saccharification process may last up to from about 24 to about 72 hours. In an embodiment a pre-saccharification step is done at 40-90 minutes at a temperature between 30-65°C, typically at about 60°C, followed by complete saccharification during fermentation in a simultaneous saccharification and fermentation step (SSF). Saccharification is typically carried out at temperatures from 30-70°C, such as 55-65°C, typically around 60°C, and at a pH between 4 and 5, normally at about pH 4.5.

The most widely used process in fermentation product production, especially ethanol production, is simultaneous saccharification and fermentation (SSF) process, in which there is no holding stage for the saccharification.

SSF may typically be carried out at a temperature between 25°C and 40°C, such as between 28°C and 36°C, such as between 30°C and 34°C, such as around 32°C, when the fermentation organism is yeast, such as a strain of *Saccharomyces cerevisiae,* and the desired fermentation product is ethanol. In an embodiment fermentation is ongoing for 6 to 120 hours, in particular 24 to 96 hours.

Other fermentation products may be fermented at conditions and temperatures, well known to the skilled person in the art, suitable for the fermenting organism in question. According to the invention the temperature may be adjusted up or down during fermentation.

In an embodiment a protease is adding during fermentation. Examples of proteases can be found in the "Proteases"-section below.

In a preferred embodiment step (ii) and step (iii) are carried out as a simultaneous saccharification and fermentation process. In such preferred embodiment the process is typically carried at a temperature between 25°C and 40°C, such as between 28°C and 36°C, such as between 30°C and 34°C, such as around 32°C. According to the invention the temperature may be adjusted up or down during fermentation.

In an embodiment simultaneous saccharification and fermentation is carried out so that the sugar level, such as glucose level, is kept at a low level such as below 6 wt.-%, preferably below about 3 wt.-%, preferably below about 2 wt.-%, more preferred below about 1 wt.-%., even more preferred below about 0.5%, or even more preferred 0.25% wt.-%, such as below about 0.1 wt.-%. Such low levels of sugar can be accomplished by simply employing adjusted quantities of enzyme and fermenting organism. A skilled person in the art can easily determine which quantities of enzyme and fermenting organism to use. The employed quantities of enzyme and fermenting organism may also be selected to maintain low concentrations of maltose in the fermentation broth. For instance, the maltose level may be kept below about 0.5 wt.-% or below about 0.2 wt.-%.

The process of the invention may be carried out at a pH in the range between 3 and 7, preferably from pH 3.5 to 6, or more preferably from pH 4 to 5.

In an embodiment a protease is adding during fermentation. Examples of proteases can be found in the "Proteases"-section below.

## Starch-Containing Materials

According to the invention sugars may be derived from starch-containing materials. Any suitable starch-containing starting material, including granular starch, may be used according to the present invention. The starting material is generally selected based on the desired fermentation product. Examples of starch-containing starting materials,

suitable for use in a process of present invention, include whole grains, corns, wheat, barley, rye, triticale, milo, sago, cassava, tapioca, sorghum, rice, peas, beans, and sweet potatoes, or mixtures thereof, or cereals, or sugar-containing raw materials, such as molasses, fruit materials, sugar cane or sugar beet, potatoes. Contemplated are both waxy and non-waxy types of corn and barley.

In a particular embodiment the starch containing material comprises maize, corn, wheat, rye, barley, triticale, sorghum, switchgrass, millet, pearl millet, foxtail millet or in a processed form such as milled corn, milled wheat, milled rye, milled barley, milled triticale, milled maize, defatted maize, defatted destarched maize, milled sorghum, milled switchgrass, milled millet, milled foxtail millet, milled pearl millet.

Particularly the starch based material comprises corn, sorghum, wheat, rye, barley, or triticale.

The term "granular starch" means raw uncooked starch, *i.e.*, starch in its natural form found in cereal, tubers or grains. Starch is formed within plant cells as tiny granules insoluble in water. When put in cold water, the starch granules may absorb a small amount of the liquid and swell. At temperatures up to 50°C to 75°C the swelling may be reversible. However, with higher temperatures an irreversible swelling called "gelatinization" begins. Granular starch to be processed may in an embodiment be a highly refined starch, preferably at least 90%, at least 95%, at least 97% or at least 99.5% pure, or it may be a more crude starch containing material comprising milled whole grain including non-starch fractions such as germ residues and fibers. The raw material, such as whole grain, is milled in order to open up the structure and allowing for further processing. Two milling processes are preferred according to the invention: wet and dry milling. In dry milling whole kernels are milled and used. Wet milling gives a good separation of germ and meal (starch granules and protein) and is often applied at locations where the starch hydrolyzate is used in production of syrups. Both dry and wet milling is well known in the art of starch processing and is equally contemplated for the process of the invention.

The starch-containing material may be reduced in particle size, preferably by dry or wet milling, in order to expose more surface area. In an embodiment the particle size is between 0.05 to 3.0 mm, preferably 0.1-0.5 mm, or so that at least 30%, preferably at least 50%, more preferably at least 70%, even more preferably at least 90% of the starch-containing material fit through a sieve with a 0.05 to 3.0 mm screen, preferably 0.1-0.5 mm screen.

## Fermenting Organisms

The term "fermenting organism" refers to any organism, including bacterial and fungal organisms, including yeast and filamentous fungi, suitable for producing a desired fermentation product. Especially suitable fermenting organisms according to the invention are able to ferment, *i.e.*, convert sugars, such as glucose, fructose, maltose, xylose, mannose and/or arabinose, directly or indirectly into the desired fermentation product. Examples of fermenting organisms include fungal organisms, such as yeast. Preferred yeast includes strains of the genus *Saccharomyces,* in particular a strain of *Saccharomyces cerevisiae* or *Saccharomyces uvarum*; a strain of *Pichia,* in particular *Pichia stipitis* or *Pichia pastoris;* a strain of the genus *Candida,* in particular a strain of *Candida utilis, Candida arabinofermentans, Candida diddensii, Candida sonorensis, Candida shehatae, Candida tropicalis,* or *Candida boidinii*. Other contemplated yeast includes strains of *Hansenula,* in particular *Hansenula polymorpha* or *Hansenula anomala*; strains of *Kluyveromyces,* in particular *Kluyveromyces marxianus* or *Kluyveromyces fagilis,* and strains of *Schizosaccharomyces,* in particular *Schizosaccharomyces pombe.*

In an embodiment the fermenting organism is a C6 sugar (hexose) fermenting organism, such as a strain of, *e.g., Saccharomyces cerevisiae.*

In connection with especially fermentation of lignocellulose derived materials, C5 sugar (pentose) fermenting organisms are also contemplated. Most C5 sugar fermenting organisms also ferment C6 sugars. Examples of C5 sugar fermenting organisms include strains of *Pichia,* such as of the species *Pichia stipitis.* C5 sugar fermenting bacteria are also known. Also some *Saccharomyces cerevisae* strains ferment C5 (and C6) sugars. Examples are genetically modified strains of *Saccharomyces spp* that are capable of fermenting C5 sugars include the ones concerned in, *e.g.,* Ho et al., 1998, Applied and Environmental Microbiology, p. 1852-1859 and Karhumaa et al., 2006, Microbial Cell Factories 5:18.

In one embodiment the fermenting organism is added in fermentation so that the viable fermenting organism, such as yeast, count per mL of fermentation medium is in the range from $10^5$ to $10^{12}$, preferably from $10^7$ to $10^{10}$, especially about $5x10^7$.

Commercially available yeast includes, *e.g.*, RED STAR™ and ETHANOL RED™ yeast (available from Fermentis/Lesaffre, USA), FALI (available from Fleischmann's Yeast, USA), SUPERSTART and THERMOSACC™ fresh yeast (available from Ethanol Technology, WI, USA), BIOFERM AFT and XR (available from NABC - North American Bioproducts Corporation, GA, USA), GERT STRAND (available from Gert Strand AB, Sweden), and FERMIOL (available from DSM Specialties).

The fermenting organism capable of producing a desired fermentation product from fermentable sugars, including glucose, fructose maltose, xylose, mannose, and/or arabinose, is preferably grown under precise conditions at a particular growth rate. When the fermenting organism is introduced into/added to the fermentation medium the inoculated fermenting organism pass through a number of stages. Initially growth does not occur. This period is referred to as the "lag phase" and may be considered a period of adaptation. During the next phase referred to as the "exponential phase" the growth rate gradually increases. After a period of maximum growth the rate ceases and the fermenting organism enters "stationary phase". After a further period of time the fermenting organism enters the "death phase" where the number of viable cells declines.

Fermentation Products

The term "fermentation product" means a product produced in a process including a fermentation step using a fermenting organism. Fermentation products contemplated according to the invention include alcohols (*e.g.*, ethanol, methanol, butanol); organic acids (*e.g.*, citric acid, acetic acid, itaconic acid, lactic acid, gluconic acid); ketones (*e.g.*, acetone); amino acids (*e.g.*, glutamic acid); gases (*e.g.*, $H_2$ and $CO_2$); antibiotics (*e.g.*, penicillin and tetracycline); enzymes; vitamins (*e.g.*, riboflavin, $B_{12}$, beta-carotene); and hormones.

In a preferred embodiment the fermentation product is ethanol, *e.g.*, fuel ethanol; drinking ethanol, *i.e.*, potable neutral spirits; or industrial ethanol or products used in the consumable alcohol industry (*e.g.*, beer and wine), dairy industry (*e.g.*, fermented dairy products), leather industry and tobacco industry. Preferred beer types comprise ales, stouts, porters, lagers, bitters, malt liquors, happoushu, high-alcohol beer, low-alcohol beer, low-calorie beer or light beer.

Fermentation

The sugars derived from plant material used in fermentation in a process of the invention may be derived from starch-containing material and/or lignocellulose-containing material. The fermentation conditions are determined based on, *e.g.*, the kind of plant material, the fermentable sugars, the fermenting organism and/or the desired fermentation product. One skilled in the art can easily determine suitable fermentation conditions. The fermentation may according to the invention be carried out at conventionally used conditions. Preferred fermentation processes are anaerobic processes.

Fermentation of Sugars Derived from Starch-Containing Materials

As mentioned above different kinds of fermenting organisms may be used for fermenting sugars derived from starch-containing material. Fermentations are often carried out using yeast, such as *Saccharomyces cerevisae,* as the fermenting organism. However, bacteria and filamentous fungi may also be used as fermenting organisms. Some bacteria have higher fermentation temperature optimum than, *e.g.*, *Saccharomyces cerevisae.* Therefore, fermentations may in such cases be carried out at temperatures as high as 75°C, *e.g.*, between 40-70°C, such as between 50-60°C. However, bacteria with a significantly lower temperature optimum down to around room temperature (around 20°C) are also known. Examples of suitable fermenting organisms can be found in the "Fermenting Organisms"-section above.

For ethanol production using yeast, the fermentation may in one embodiment go on for 24 to 96 hours, in particular for 36 to 72 hours. In an embodiment the fermentation is carried out at a temperature between 20 to 40°C, preferably 28 to 36°C, in particular around 32°C. In an embodiment the pH is from pH 3 to 6, preferably around pH 4 to 5.

Especially contemplated is simultaneous hydrolysis/saccharification and fermentation (referred to as "SSF") where there is no separate holding stage for the hydrolysis/saccharification, meaning that the hydrolysing enzyme(s), the fermenting organism(s), and calmodulin protein may be added together. However, it should be understood that the calmodulin protein may also be added separately. When fermentation is performed simultaneous with saccharification (i.e., SSF) the temperature is preferably between 20 to 40°C, preferably 28 to 36°C, in particular around 32°C when the fermentation organism is a strain of *Saccharomyces cerevisiae* and the desired fermentation product is ethanol.

Other fermentation products may be fermented at temperatures known to the skilled person in the art to be suitable for the fermenting organism in question.

The process of the invention may be performed as a batch or as a continuous process. The fermentation process of the invention may be conducted in an ultrafiltration system where the retentate is held under recirculation in the presence of solids, water, and the fermenting organism, and where permeate is the desired fermentation product containing liquid. Equally contemplated if the process is conducted in a continuous membrane reactor with ultrafiltration membranes and where the retentate is held under recirculation in presence of solids, water, the fermenting organism and where the permeate is the fermentation product containing liquid.

After fermentation the fermenting organism may be separated from the fermented slurry and recycled.

Fermentations are typically carried out at a pH in the range between 3 and 7, preferably from pH 3.5 to 6, such as around pH 5. Fermentations are typically ongoing for 24-96 hours.

**Methods and processes according to the invention**

The main aspect according to the present invention relates to processes for hydrolysing starch containing material to sugars and subsequently fermenting the sugars to alcohol, particularly ethanol, wherein a GH5 xylanase of subfamily 35 is present during the hydrolysis. Different processes and process conditions have been described above.

A particular aspect of the invention relates to a process for producing fermentation products from starch-containing material comprising the steps of:

a) liquefying starch-containing material using an alpha-amylase:
b) saccharifying the liquefied material using a glucoamylase;
c) fermenting using a fermenting organism, wherein saccharification is carried out in the presence of a GH5 xylanase, wherein the GH5 xylanase is selected from subfamily 35.

In a particular embodiment the starch containing material comprises maize, corn, wheat, rye, barley, triticale, sorghum, switchgrass, millet, pearl millet, foxtail millet or in a processed form such as milled corn, milled wheat, milled rye, milled barley, milled triticale, milled maize, defatted maize, defatted destarched maize, milled sorghum, milled switchgrass, milled millet, milled foxtail millet, milled pearl millet.

Particularly the starch based material comprises corn, sorghum, wheat, rye, barley, or triticale.

The GH5 xylanases are particularly effective on highly branched xylans and thus in one embodiment the starch-containing material comprises highly branched xylan.

The GH5 xylanases are preferably selected from the group consisting of subfamily 21, 34, or 35.

In one specific embodiment the subfamily 35 GH5 xylanase is selected from the xylanase shown as amino acids 37 to 573 of SEQ ID NO: 2, or a GH5 xylanase having at least 75%, at least 80%, at least 85%, at least 90%, at least 95%, at least 96%, at least 97%, at least 98%, at least 99% identity to amino acids 37 to 573 of SEQ ID NO: 2.

In another specific embodiment the subfamily 35 GH5 xylanase is selected from the xylanase shown as amino acids 36 to 582 of SEQ ID NO: 4, or a GH5 xylanase having at least 75%, at least 80%, at least 85%, at least 90%, at least 95%, at least 96%, at least 97%, at least 98%, at least 99% identity to amino acids 36 to 582 of SEQ ID NO: 4.

The fermentation product is in one embodiment an alcohol, particularly ethanol.

Saccharification and fermentation may be performed separately or simultaneously. The fermenting organism is preferably a *Saccharomyces* sp., more particularly *Saccharomyces cerevisiae.* Suitable commercial yeast strain available have been described in the section "Fermenting organisms" herein.

The present invention is further described by the following examples.

**Examples**

**Media and Solutions**

Preparation of Destarched Maize (DSM)

107 kg of milled maize (<10 mm) was mixed in a tank with 253 kg of tap water at 53°C to make a slurry. The temperature of the slurry was 47°C and the pH 5.9. The pH was adjusted to 6.15 with 1 L of 1 N NaOH and the tank was then heated to 95°C. 1.119 kg of Termamyl® alpha-amylase (Novozymes A/S, Bagsvaerd, Denmark) was added at 52°C and incubated for 80 minutes at 95°C. The pH measured at the end of the incubation was 6.17. Cold tap water was added to the slurry and the slurry was centrifuged and decanted 3 times using a Westfalia decanter CA-225-110 (4950±10 rpm, flow ~600l/h) giving 64.5kg of sludge. The sludge was then collected, frozen and freeze-dried to give 17.1 kg of destarched maize (DSM).

Preparation of Defatted Destarched Maize (DFDSM)

500mL acetone was added to 100 gram of destarched maize, prepared as described above. The slurry was stirred for 5 minutes and allowed to settle. The acetone was decanted and the procedure was repeated 2 times. The residue was air dried overnight to give defatted destarched maize (DFDSM) which was stored at room temperature.

Preparation of Destarched Sorghum

Whole sorghum seeds were milled and sieved and a fraction below 0.5 mm was used for further processing. The sieved fraction was suspended in 25 mM NaOAc pH 5.5 at 20 % dry matter and destarched. The destarching involved a first step at 85 °C with 500 ppm Termamyl SC alpha-amylase (Novozymes A/S, Bagsvaerd, Denmark) for 20

min followed by an overnight incubation using 250 ppm Attenuzyme Flex (Novozymes A/S, Bagsvaerd, Denmark) at 65 °C. The slurry was centrifuged and the liquid decanted. After this another destarching was made using by adding MilliQ water and 200 ppm Termamyl SC and 200 ppm Attenuzyme Flex and incubating overnight at 65 °C.

The sorghum fiber was separated from the liquid by vacuum filtration through a Whatman F glass fiber filter. The filter cake was then washed several times with excess of water to remove soluble sugars. Finally the destarched sorghum fiber was dried in an oven at 65 °C and the dry fiber milled quickly in a coffee grinder so that the particle size was in general less than 1mm.

Preparation of corn fiber gum

Corn destarching

107 kg pre-milled corn (<1.0 mm) mixed with 253 kg water at 53 °C. The mixture was heated to 95 °C and the pH adjusted to 6.2 with 1 M NaOH. 1.12 kg, Termamyl 120 L (a commercial alpha amylase available from Novozymes A/S) was added and the reaction was kept around 90 °C for 3 hrs. After 3 hrs, cold water was added to a total reaction weight of 600 kg. Liquid solid separation was done with a Westfalia decanter, CA-225-110, 4950 rpm, flow 600 L/hour. The solid fiber fraction was re-slurried and separated twice with water to a total weight of 600 kg followed by separation as described above. The final fiber fraction was divided into smaller portions and freeze dried for 3-4 days.

Termamyl 120 L is a *Bacillus licheniformis* alpha amylase, however, other acid alpha amylases showing good temperature stability may also be used. Examples include, a *Bacillus stearothermophilus* alpha-amylases, which have a double deletion corresponding to delta(181-182) and further comprise a N193F substitution (also denoted I181* + G182* + N193F) compared to the wild-type BSG alpha-amylase amino acid sequence set forth in SEQ ID NO: 3 disclosed in WO 99/19467.

Corn Fiber Gum extraction

20 g of destarched corn was added to 200 mL boiling MilliQ™ water together with 0.8 g of NaOH and 0.8 g of Ca(OH)2. The mixture was kept for 1 hour at 96 C and then centrifuged for 20 min at 6000 g.

The supernatant looked milky and fatty and therefore fats were extracted by shaking with hexane (1 part hexane to 4 parts corn fiber gum). After a resting time the hexane was removed by pipetting.

A 2nd extraction (CFG2) was made by dissolving the pellet in 200 mL 1 M NaOH. The mixture was kept for 1 hour at 96 C and then centrifuged for 20 min at 6000 g and the supernatant adjusted to pH 6 with 4 M HCl.

Both CFG fractions were concentrated using a rotavap and precipitated in EtOH at a final concentration of 90 %. The pellet from the 0.1 M NaOH will from now on be called CFG1. Due to the high pH in 1 M NaOH extract (CFG2) this fraction was dialysed on a 2 L measuring cylinder with deionised water with the tap dripping overnight using a 3 kDa dialysis membrane.

Before application trials both CFG1 and CFG2 were centrifuged at 25000 g for 25 min and filtered through a 0.44 μm syringe filter. The dry matter in CFG2 fractions was determined with a Mettler Toledo HR73

## Assays

Xylose assay

A xylose standard curve from 0 to 125 μg xylose/mL was prepared from a stock solution of 2.5 mg xylose/mL (prepared by dissolving 0.125 g xylose in 50 mL de-ionised water).

Assay principle. The interconversion of the α- and β-anomeric forms of D-xylose is catalysed by xylose mutarotase (XMR) using the D-xylose assay kit from Megazyme International Ireland. The β-D-xylose is oxidised by NAD+ to D-xylonic acid in the presence of ß-xylose dehydrogenase (β-XDH) at pH 7.5. The amount of NADH formed in this reaction is stoichiometric with the amount of D-xylose and is measured by the increase in absorbance at 340 nm.

$$\alpha\text{-D-Xylose} \xleftrightarrow{(\text{XMR})} \beta\text{-D-xylose}$$

$$\beta\text{-D-Xylose} + \text{NAD}^{+} \xrightarrow{(\beta\text{-XDH})} \text{D-xylonic acid} + \text{NADH} + \text{H}^{+}$$

**Example 1: Cloning of GH5 xylanases**

Donor strains

The xylanase from *Acetivibrio cellulolyticus* CD2 was identified in part of its public genome sequence as originally published under the accession number UniProt: E1KC96 (Lucas S., Copeland A., Lapidus A., Cheng J.-F., Bruce D., Goodwin L.,Pitluck S., Land M.L., Hauser L., Chang Y.-J., Jeffries C., Mouttaki H., He Z., Zhou J., Hemme C.L., Woyke T.J.; "The draft genome of Acetivibrio cellulolyticus CD2."; Submitted (AUG-2010) to the EMBL/GenBank/DDBJ databases). The xylanase has also been published in Hemme CL, Mouttaki H, Lee YJ, Zhang G, Goodwin L, Lucas S, Copeland A, Lapidus A, Glavina del Rio T, Tice H, et al.: Sequencing of multiple clostridial genomes related to biomass conversion and biofuel production. J Bacteriol 2010, 192(24):6494-6496. Uniprot: E1KC96 is SEQ ID NO: 7 and SEQ ID NO:8 are both GH5_34 xylanses.

The xylanase from the strains *Paenibacillus* sp. 18054 (SEQ ID NO: 3 and 4) and *Paenibacillus illinoisensis* (SEQ ID NO: 1 and 2) were identified by shotgun genome sequencing. Both xylanases belong to family GH5_35. The strains were isolated from a thermal sample from New Zealand in 1991. *P. illionensis* is deposited as DSMZ under accession number DSM16232.

The elephant dung metagenome xylanase (SEQ ID NO: 5 and 6) belongs to GH5_21 family was obtained by deep sequencing of a metagenome extract. The dung of a six years old female Asian elephant (name "Kandy") living in the zoological garden in Hamburg, Germany was used. The DNA isolation was performed with the QIAamp DNA Stool kit from Qiagen (Hilden, Germany) as described in the manufacturer's protocol.

Genome sequencing, the subsequent assembly of reads and the gene discovery (i.e. annotation of gene functions) is known to the person skilled in the art and the service can be purchased commercially.

Cloning examples

Based on the nucleotide sequences mentioned in section/example "Donor strains", one codon optimized synthetic gene per xylanase was synthesized and purchased commercially. A truncated tri modular version of Uniprot: E1KC96 ($A^{31}$-$P^{650}$) was cloned and is from here on referred to as SEQ ID NO: 7.

The optimization process and the cloning of the purchased synthetic genes were subcloned using ClaI and MluI restriction sites into a Bacillus expression vector as described in WO 12/025577. The xylanases were expressed with a *Bacillus clausii* secretion signal (BcSP; with the following amino acid sequence: MKKPLGKIVASTALLISVAFSSSIASA, originating from the protease AprH of B. *clausii*). BcSP replaced all native secretion signals respectively in all genes.

Downstream of the BcSP sequence an affinity tag sequence was introduced to ease the purification process (Histag; with the following amino acid sequence: HHHHHHPR for the xylanase from elephant dung metagenome, *Paenibacillus* sp. 18054 and *Acetivibrio cellulolyticus* and HQHQHQHPR for the *Paenibacillus illinoisensis* xylanase, respectively). The gene that was expressed therefore comprised the BcSP sequence followed by the Histag sequence followed by the mature wild type xylanase sequence

The final expression plasmids (BcSP-Histag-xylanase) were individually transformed into a *Bacillus subtilis* expression host. The xylanase BcSP-fusion genes were integrated by homologous recombination into the *Bacillus subtilis* host cell genome upon transformation.

The gene construct was expressed under the control of a triple promoter system (as described in WO 99/43835). The gene coding for chloramphenicol acetyltransferase was used as maker (as described in (Diderichsen et al., 1993, Plasmid 30: 312-315). Transformants were selected on LB media agar supplemented with 6 microgram of chloramphenicol per ml. One recombinant *Bacillus subtilis* clone containing the respective xylanase expression construct was selected and was cultivated on a rotary shaking table in 500 ml baffled Erlenmeyer flasks each containing 100 ml yeast extract-based media. After 3-5 days cultivation time at 30 °C to 37°C, enzyme containing supernatants were harvested by centrifugation and the enzymes were purified by His-tag purification.

**Example 2: Purification of GH5 xylanases**

All His-tagged enzymes were purified by immobilized metal chromatography (IMAC) using $Ni^{2+}$ as the metal ion on 5 mL HisTrap Excel columns (GE Healthcare Life Sciences). The purification took place at pH 8 and the bound proteins were eluted with imidazole. The purity of the purified enzymes was checked by SDS-PAGE and the concentration of each enzyme determined by Abs 280 nm after a buffer exchange.

**Example 3: Measurement of soluble and insoluble dietary fiber in the substrate defatted de-starched maize (DFDSM) and correlation to soluble xylose measured after enzymatic incubation**

400 mg of defatted de-starched maize (DFDSM) was added to NaOAc-buffer (5 mL, pH 5). The mixture was heated to between 90-100°C, then Termamyl 300 DX (100 μL, Novozymes A/S, Bagsvaerd, Denmark) was added and the mixture was incubated for 1 hr. The mixture was then cooled and amyloglucosidase from *Aspergillus niger* (500 μL, catalogue number E-AMGDF, for use in Megazyme Total Starch and Dietary Fiber, Megazyme International Ireland, Wicklow, Ireland) was added and samples were incubated overnight (16 h) at 60°C. The mixture was then cooled and centrifuged at 2500 x g for 10 min at 5°C. The supernatant was collected and NaOAc-buffer (5 mL, pH 5) was added to the residue and centrifuged at 2500 x g, 10 min, 5°C. This procedure was repeated twice. The supernatants were then collected, pooled and analysed for soluble NSP as described in A. The residue was analysed for insoluble NSP as described in B.

A: Soluble NSP, supernatant

The pooled supernatants were diluted to a fixed volume from which a 5 mL aliquot of supernatant was taken. To this aliquot was added 20.1 mL cold 99.9 % ethanol and the mixture was kept on ice for approx. 15 min for precipitation of polymers with a DP>10. After centrifuging at 2500 x g, 5 °C for 10 min, the supernatant was discarded.

5 mL cold 80% ethanol was added to the pellet and the mixture was kept on ice for approx. 15 min. After centrifuging at 2500 x g, 5 °C for 10 min, the supernatant was discarded.

Acid hydrolysis of the precipitate was conducted by the addition of MQ water (7.9 mL), myoinositol (0.5 mL, internal standard) and 12M $H_2SO_4$ (0.3 mL) and autoclaving at 125 °C for 55 minutes.

B: Insoluble NSP, residue

The pellet obtained after AMG treatment was hydrolysed by the addition of MQ water (74 mL), myoinositol (10 mL, internal standard) and 12M $H_2SO_4$ (3 mL) and autoclaving at 125°C for 55 minutes.

GLC Analysis

After autoclaving, the samples were reduced with borohydride to produce alditol sugars and these were derivatised by acetylation to become volatile for GLC analyses on an instrument with FID detector (Pettersson et al, (1995) "Total dietary fiber determined as neutral sugar residues, uronic acid residues, and Klason lignin (the Uppsala method), Collaborative study", J. AOAC Int. 78:1030-1044). The concentration of the soluble or insoluble sugars was determined relative to myo-inositiol.

Percentage solubilised xylose

When DFDSM is incubated with enzyme at 40°C for 4 hours, the enzyme solubilizes the xylan in the substrate and this solubilized xylan is then hydrolysed further by acid. The xylose released is measured spectrophotometrically using a D-xylose assay kit (Megazyme, catalogue number K-xylose). This xylose (which is actually enzyme solubilized xylan) is then correlated to the amount of total xylose of the substrate measured by GLC as described above.

The DFDSM contains 99 % insoluble and 1 % soluble xylose, in total 14.81 % xylose which represents the concentration of xylose polymer (DP>10) present in the sample (DFDSM) according to the analysis. Based on the release of xylose measured by the Megazyme kit which calculates release based on sample weight, the amount of xylose released can be calculated as follows: e.g. 1 % release from 400 mg of sample equals 4 mg of xylose. In 400 mg sample there is 400mg x 14.81 % xylose, equal to 59.22 mg xylose. The gross xylose (insoluble + soluble) release is that case 4 mg/59.22 mg which represents a release of 6.75 mg xylose, but it should be noted that this value must be corrected for the passive release obtained for the non-enzyme supplemented control. This corrected value is defined herein as the percentage solubilised xylose.

**Example 4: Hydrolysis of Defatted Destarched Maize (DFDSM) with GH5 xylanases**

Defatted destarched maize (DFDSM, 400mg) was added to aqueous sodium acetate (0.1 M, 3.9 mL) solution containing calcium chloride (5 mM) at pH 5 and the mixture heated to 40°C for 30 minutes. 100μL buffer or enzyme solution was added and the sample was heated at 40°C for 4 hours. The sample was cooled to 5°C and centrifuged (4000 rpm, 5°C) for 10 minutes. 1.7 mL of the sample was transferred to an Eppendorf tube and the enzyme deactivated by heating to 95°C for 10 minutes. The samples were then frozen until hydrolyzed.

The supernatant was thawed and centrifuged (14000 rpm) for 5 minutes. The supernatant (250 μL) was diluted with Milli-Q water (250 μL) in glass tubes and HCl (1.63 M, 2.0 mL) was added. The reaction was heated to 100°C for 1 hour then cooled in an ice bath. Aqueous NaOH solution (1.3 M, 2.5 mL) was added whilst the samples were cooled on ice and the samples were stored at 0-5°C whilst xylose content was analysed using the xylose assay. The results are presented in tables 2, 3 and 4.

Table 2: Xylose release from DFDSM using GH5 xylanase SEQ ID NO: 2

Table 2 shows the amount of xylose measured after acid hydrolysis of supernatants (% of dry matter and % solubilized xylose of total xylose) when incubating defatted de-starched maize (DFDSM) with the GH5 xylanase of SEQ ID NO: 2 at different enzyme concentrations compared to blank and the commercial GH11 xylanase Ronoxyme WX.

| GH5 Xylanase | Conc. [mg EP/kg] | Soluble xylose (%) | % solubilised xylose[1] | Significance[2] | Std. Dev. |
|---|---|---|---|---|---|
| Blank | 0 | 0.039 | 0.3 | C | 0.004 |
| Ronozyme WX (GH11) | 25 | 0.101 | 0.7 | C | 0.009 |
| SEQ ID NO: 2 | 10 | 0.903 | 6.2 | B | 0.116 |
| SEQ ID NO: 2 | 25 | 1.295 | 9.0 | A | 0.225 |
| SEQ ID NO: 2 | 50 | 1.200 | 8.2 | A | 0.133 |

[1]Percentage solubilised xylose was calculated as described in example 3.
[2]ABC: Least squared values within a column not sharing a capital letter differ significantly (P<0.05 all pairs Tukey-Kramer HSD).

Table 3: Xylose release from DFDSM using GH5 xylanase SEQ ID NO: 4

Table 3 shows the amount of xylose measured after acid hydrolysis of supernatants (% of dry matter and % solubilized xylose of total xylose) when incubating defatted de-starched maize (DFDSM) with the GH5 xylanase of SEQ ID NO: 4 at different enzyme concentrations compared to blank and the commercial GH11 xylanase Ronoxyme WX.

| GH5 Xylanase | Conc. [mg EP/kg] | Soluble xylose (%) | % solubilised xylose[1] | Significance[2] | Std. Dev. |
|---|---|---|---|---|---|
| Blank | 0 | 0.040 | 0.3 | C | 0.001 |
| Ronozyme WX (GH11) | 25 | 0.101 | 0.7 | C | 0.010 |
| SEQ ID NO: 4 | 10 | 0.890 | 5.9 | B | 0.018 |
| SEQ ID NO: 4 | 25 | 1.016 | 7.0 | B | 0.078 |
| SEQ ID NO: 4 | 50 | 1.340 | 9.2 | A | 0.280 |

[1]Percentage solubilised xylose was calculated as described in example 3.
[2]ABC: Least squared values within a column not sharing a capital letter differ significantly (P<0.05 all pairs Tukey-Kramer HSD).

Table 4: Xylose release from DFDSM using GH5 xylanase SEQ ID NO: 18

Table 4 shows the amount of xylose measured after acid hydrolysis of supernatants (% of dry matter and % solubilized xylose of total xylose) when incubating defatted de-starched maize (DFDSM) with the GH5 xylanase of SEQ ID NO: 6 at different enzyme concentrations compared to blank and the commercial GH11 xylanase Ronoxyme WX.

| GH5 Xylanase | Conc. [mg EP/kg] | Soluble xylose (%) | % solubilised xylose[1] | Significance[2] | Std. Dev. |
|---|---|---|---|---|---|
| Blank | 0 | 0.001 | 0.0004 | C | 0.001 |

(continued)

| Table 4 shows the amount of xylose measured after acid hydrolysis of supernatants (% of dry matter and % solubilized xylose of total xylose) when incubating defatted de-starched maize (DFDSM) with the GH5 xylanase of SEQ ID NO: 6 at different enzyme concentrations compared to blank and the commercial GH11 xylanase Ronoxyme WX. | | | | | |
|---|---|---|---|---|---|
| **GH5 Xylanase** | **Conc. [mg EP/kg]** | **Soluble xylose (%)** | **% solubilised xylose[1]** | **Significance[2]** | **Std. Dev.** |
| Ronozyme WX (GH11) | 25 | 0.053 | 0.04 | C | 0.009 |
| SEQ ID NO: 6 | 10 | 0.934 | 6.4 | B | 0.154 |

[1]Percentage solubilised xylose was calculated as described in example 3.
[2]ABC: Least squared values within a column not sharing a capital letter differ significantly (P<0.05 all pairs Tukey-Kramer HSD).

As can be seen from tables 2, 3 and 4, the GH5 xylanases are significantly better at releasing xylose from defatted de-starched maize than either blank of the commercial GH11 xylanase Ronoxyme WX.

**Example 5: Enzymatic preparation of corn fiber gum hydrolysates**

The purified enzymes used for the hydrolysis was SEQ ID NO: 2, SEQ ID NO: 4, SEQ ID NO: 6, SEQ ID NO: 7, SEQ ID NO: 8 (pentamodular GH5_34 from *Clostridium thermocellum* NzyTech (Catalogue Number (SKU): CR0061, Correia et al. J. Biol. Chem. 2011, 286:22510-22520)), Pentopan mono, Pulpzyme, and Shearzyme. For the commercial products the main xylanase was purified product by anion exchange chromatography and tested.

All reactions were performed with CFG1 at 3.1 % DM as the final concentration. The assay also contained 45 mM MES buffer pH6 and the final enzyme concentrations tested were: 41, 18, 7.3, and 1.8 mg/L for 2 hours and 41 mg/L for 24 hrs at 40 °C for most enzymes. SEQ ID NO: 7 was assayed at 30 °C due to low thermostability. All reactions were stopped at 95 °C for 10 min and analyzed by HPLC-SEC on an ICS-3000 with RI detection (Dionex). The columns used were a PWXL guard column and a PWXL-3000 and -5000 (Tosoh) connected in series. The eluent was 50 mM NaOAc pH 5 and the flow rate 0.5 ml/min. retention times were compared to a pullulan molecular weight standard between 803-1.3 kDa and glucose, maltotriose and maltopentaose.

**Results**

In the following tables the characteristics of the corn fiber gum and the respective products made by enzymatic treatment are given.

Table 5. Characteristics of the alkali solubilized corn fiber gum.

| Mp (Da) | Mn (Da) | Mw (Da) | Polydispersity |
|---|---|---|---|
| 227492 | 129701 | 302344 | 2.3 |

Table 6. Characteristics of corn fiber gum hydrolysates made using the main GH11 xylanase activity in Pentopan Mono™. The calculations are based on the fraction above DP2.

| Enzyme conc. (mg/L) | Incubation time (hrs) | Mp (Da) | Mn (Da) | Mw (Da) | Polydispersity |
|---|---|---|---|---|---|
| 1.8 | 2 | 228839 | 124375 | 290617 | 2.34 |
| 7.3 | 2 | 184165 | 117007 | 263390 | 2.25 |
| 18 | 2 | 162696 | 117212 | 257179 | 2.19 |
| 41 | 2 | 205511 | 113496 | 254255 | 2.24 |
| 41 | 24 | 209217 | 115376 | 243574 | 2.11 |

Table 7. Characteristics of corn fiber gum hydrolysates made using the main GH11 xylanase activity in Pulpzyme™. The calculations are based on the fraction above DP2.

| Enzyme conc. (mg/L) | Incubation time (hrs) | Mp (Da) | Mn (Da) | Mw (Da) | Polydispersity |
|---|---|---|---|---|---|
| 1.8 | 2 | 217344 | 126520 | 285321 | 2.26 |
| 7.3 | 2 | 207962 | 124297 | 265849 | 2.14 |
| 18 | 2 | 189005 | 117782 | 249998 | 2.12 |
| 41 | 2 | 193856 | 119805 | 251758 | 2.10 |
| 41 | 24 | 211270 | 115520 | 245405 | 2.12 |

Table 8. Characteristics of corn fiber gum hydrolysates made using the main GH10 xylanase activity in Shearzyme™ 500 L. The calculations are based on the fraction above DP2.

| Enzyme conc. (mg/L) | Incubation time (hrs) | Mp (Da) | Mn (Da) | Mw (Da) | Polydispersity |
|---|---|---|---|---|---|
| 1.8 | 2 | 212907 | 105298 | 246478 | 2.34 |
| 7.3 | 2 | 208994 | 84282 | 221618 | 2.63 |
| 18 | 2 | 197932 | 74413 | 205479 | 2.76 |
| 41 | 2 | 194088 | 69845 | 197069 | 2.82 |
| 41 | 24 | 174418 | 44927 | 160978 | 3.58 |

Table 9. Characteristics of corn fiber gum hydrolysates made using the family GH5_35 xylanase of SEQ ID NO: 2. The calculations are based on the fraction above DP2.

| Enzyme conc. (mg/L) | Incubation time (hrs) | Mp (Da) | Mn (Da) | Mw (Da) | Polydispersity |
|---|---|---|---|---|---|
| 1.8 | 2 | 166060 | 20266 | 132629 | 6.54 |
| 7.3 | 2 | 63011 | 13211 | 70669 | 5.35 |
| 18 | 2 | 38557 | 9821 | 46983 | 4.78 |
| 41 | 2 | 21607 | 8057 | 35598 | 4.42 |
| 41 | 24 | 8701 | 6348 | 24976 | 3.92 |

Table 10. Characteristics of corn fiber gum hydrolysates made using the family GH5_35 xylanase of SEQ ID NO: 4. The calculations are based on the fraction above DP2.

| Enzyme conc. (mg/L) | Incubation time (hrs) | Mp (Da) | Mn (Da) | Mw (Da) | Polydispersity |
|---|---|---|---|---|---|
| 1.8 | 2 | 172666 | 22324 | 145242 | 6.51 |
| 7.3 | 2 | 63280 | 12591 | 70743 | 5.62 |
| 18 | 2 | 39090 | 9358 | 47688 | 5.1 |
| 41 | 2 | 20815 | 7618 | 35496 | 4.66 |
| 41 | 24 | 8837 | 5722 | 22324 | 3.9 |

Table 11. Characteristics of corn fiber gum hydrolysates made using the family GH5_21 xylanase of SEQ ID NO: 6. The calculations are based on the fraction above DP2.

| Enzyme conc. (mg/L) | Incubation time (hrs) | Mp (Da) | Mn (Da) | Mw (Da) | Polydispersity |
|---|---|---|---|---|---|
| 1.8 | 2 | 8228 | 8368 | 28884 | 3.45 |
| 7.3 | 2 | 4937 | 4709 | 14995 | 3.18 |
| 18 | 2 | 4735 | 4007 | 12885 | 3.22 |
| 41 | 2 | 2318 | 3645 | 10920 | 3.00 |
| 41 | 24 | 2268 | 2864 | 8559 | 2.99 |

Table 12. Characteristics of corn fiber gum hydrolysates made using the family GH5_34 xylanase of SEQ ID NO: 7. The calculations are based on the fraction above DP2.

| Enzyme conc. (mg/L) | Incubation time (hrs) | Mp (Da) | Mn (Da) | Mw (Da) | Polydispersity |
|---|---|---|---|---|---|
| 1.8 | 2 | 193478 | 74799 | 234258 | 3.13 |
| 7.3 | 2 | 82734 | 24762 | 137377 | 5.55 |
| 18 | 2 | 47096 | 14798 | 78416 | 5.30 |
| 41 | 2 | 16107 | 8499 | 41935 | 4.93 |
| 41 | 24 | 4541 | 4390 | 16530 | 3.77 |

Table 13. Characteristics of corn fiber gum hydrolysates made using the family GH5_34 pentamodular arabinoxylanase of SEQ ID NO: 8 (available from NzyTech). The calculations are based on the fraction above DP2.

| Enzyme conc. (mg/L) | Incubation time (hrs) | Mp (Da) | Mn (Da) | Mw (Da) | Polydispersity |
|---|---|---|---|---|---|
| 1.8 | 2 | 137128 | 54827 | 229844 | 4.19 |
| 7.3 | 2 | 77794 | 31050 | 153021 | 4.93 |
| 18 | 2 | 38688 | 11646 | 83398 | 7.16 |
| 41 | 2 | 8894 | 7192 | 38017 | 5.29 |
| 41 | 24 | 2533 | 2703 | 10810 | 4.00 |

The corn fiber gum hydrolysate data clearly showed that all the tested GH5 xylanases from subfamilies GH5_21, GH5_34 and GH5_35 have the capacity to degrade this highly substituted xylan. The DP1-DP2 fraction was below 1 % under all tested conditions.

The two family GH11 xylanases tested did not degrade the xylan at all while the GH10 xylanase present in Shearzyme 500 L only had moderate corn fiber gum-degrading capacity.

Based on the above results the effects of adding GH5 xylanases in a starch to ethanol process was tested.

**Example 6: Ethanol yield improvement using a GH5_35 and GH5_21 xylanase added in saccharification/fermentation**

Liquefaction of a mash from milled corn was performed at 85°C using a commercial alpha amylase, Liquozyme™ SCDS (available from Novozymes A/S) according to the table below.

| Mash | Enzyme(s)/ kg/ t DS | Temp. °C | Time Min. | DS % | pH | Mixing Rpm | Calcium ppm |
|---|---|---|---|---|---|---|---|
| | Corn: 0,3 kg SCDS/ t DS | 85 | 120 | 28 | 5,5 | 80 after 20min at 300 | 30 |

The liquefied mash was split into three parts and simultaneous saccharification and fermentation performed according to the below table. A commercial glucoamylase product, Spirizyme Excel™ (available from Novozymes A/S) was used for saccharification and the yeast was Ethanol Red™ (available from Lallemand). Fermentation was followed by weight loss measured twice a day and by HPLC at the end of fermentation. The control sample only contained the glucoamylase enzymes. In addition one sample contained glucoamylase and the GH5_35 xylanase (U2AGD/SEQ ID NO: 4) and another sample contained glucoamylase and the GH5_21 xylanase (U2C9W/SEQ ID NO: 6). The ethanol yield is shown in Figure 1 and demonstrates that the addition of a GH5 xylanase increased the ethanol yield compared to the control. In particular the GH5_35 xylanase resulted in an increase of around 2%.

| Sample | Enzyme(s) | T (°C) | Time (Hours) | pH | Yeast (mill/ml) |
|---|---|---|---|---|---|
| Control | 0.55 kg Spirizyme Excel/ t DS | 32 | 96 | 4.5 | 30 |
| GH5_35 | 0.55 kg Spirizyme Excel/ t DS + 50 µg SEQ ID NO: 4 / g DS | 32 | 96 | 4.5 | 30 |
| GH5_21 | 0.55 kg Spirizyme Excel/ t DS + 50 µg SEQ ID NO: 6 / g DS | 32 | 96 | 4.5 | 30 |

## Claims

1. A process for producing fermentation products from starch-containing material comprising the steps of:

   a) liquefying starch-containing material using an alpha-amylase:
   b) saccharifying the liquefied material using a glucoamylase;
   c) fermenting using a fermenting organism, wherein saccharification is carried out in the presence of a GH5 xylanase, wherein the GH5 xylanase is selected from subfamily 35.

2. The process according to claims 1, wherein the starch containing material comprises maize, corn, wheat, rye, barley, triticale, sorghum, switchgrass, millet, pearl millet, foxtail millet or in a processed form such as milled corn, milled wheat, milled rye, milled barley, milled triticale, milled maize, defatted maize, defatted destarched maize, milled sorghum, milled switchgrass, milled millet, milled foxtail millet, milled pearl millet.

3. The process according to any of the preceding claims, wherein the starch-containing material comprises highly branched xylan.

4. The process according to claim 1, wherein the subfamily 35 GH5 xylanase is selected from the xylanases shown as amino acids 37 to 573 of SEQ ID NO: 2, amino acids 36 to 582 of SEQ ID NO: 4, or a xylanase having at least 75% identity to amino acids 37 to 573 of SEQ ID NO: 2, or amino acids 36 to 582 of SEQ ID NO: 4.

5. The process according to any of the preceding claims, wherein the plant material comprises corn, sorghum, wheat, rye, barley, or triticale.

6. The process according to any of the preceding claims, wherein the fermentation product is alcohol, particularly ethanol.

7. The process according to any of the preceding claims, wherein the fermenting organism is yeast, particularly *Saccharomyces* sp., more particularly *Saccharomyces cerevisiae.*

8. The process according to any of the preceding claims, wherein steps b) and c) are performed simultaneously.

9. A use of a GH5 subfamily 35 xylanase for producing ethanol from starch containing material.

10. The use according to claim 9, wherein starch containing material comprises corn, sorghum, wheat, rye, barley, or triticale.

**Patentansprüche**

1. Verfahren zum Herstellen von Fermentationsprodukten aus stärkehaltigem Material, umfassend die Schritte:

   a) Verflüssigen von stärkehaltigem Material unter Verwendung einer alpha-Amylase;
   b) Verzuckern des stärkehaltigem Materials unter Verwendung einer Glucoamylase;
   c) Fermentieren unter Verwendung eines fermentierenden Organismus, wobei Verzuckerung in der Gegenwart einer GH5-Xylanase ausgeführt wird, wobei die GH5-Xylanase aus Unterfamilie 35 ausgewählt ist.

2. Verfahren nach Ansprüchen 1, wobei das stärkehaltige Material Mais, Korn, Weizen, Roggen, Gerste, Triticale, Sorghum, Rutenhirse, Hirse, Perlhirse, Kolbenhirse, oder in einer prozessierten Form wie gemahlenes Korn, gemahlener Weizen, gemahlener Roggen, gemahlene Gerste, gemahlener Triticale, gemahlener Mais, entfetteter Mais, entfetteter entstärkter Mais, gemahlenes Sorghum, gemahlene Rutenhirse, gemahlene Hirse, gemahlene Kolbenhirse, gemahlene Perlhirse umfasst.

3. Verfahren nach einem beliebigen der voranstehenden Ansprüche, wobei das stärkehaltige Material hoch verzweigtes Xylan umfasst.

4. Verfahren nach Anspruch 1, wobei die Unterfamilie 35-GH5-Xylanase aus den als Aminosäuren 37 bis 573 von SEQ ID NO: 2, Aminosäuren 36 bis 582 von SEQ ID NO: 4 gezeigten Xylanasen, oder einer Xylanase mit mindestens 75% Identität zu Aminosäuren 37 bis 573 von SEQ ID NO: 2, oder Aminosäuren 36 bis 582 von SEQ ID NO: 4 ausgewählt ist.

5. Verfahren nach einem beliebigen der voranstehenden Ansprüche, wobei das Pflanzenmaterial Mais, Sorghum, Weizen, Roggen, Gerste oder Triticale umfasst.

6. Verfahren nach einem beliebigen der voranstehenden Ansprüche, wobei das Fermentationsprodukt Alkohol ist, insbesondere Ethanol.

7. Verfahren nach einem beliebigen der voranstehenden Ansprüche, wobei der fermentierende Organismus Hefe ist, insbesondere *Saccharomyces* sp., stärker insbesondere *Saccharomyces cerevisiae.*

8. Verfahren nach einem beliebigen der voranstehenden Ansprüche, wobei Schritte b) und c) gleichzeitig durchgeführt werden.

9. Verwendung einer GH5-Unterfamilie 35-Xylanase zum Herstellen von Ethanol aus stärkehaltigem Material.

10. Verwendung nach Anspruch 9, wobei stärkehaltiges Material Mais, Sorghum, Weizen, Roggen, Gerste oder Triticale umfasst.

**Revendications**

1. Procédé de production de produits de fermentation à partir d’une matière contenant de l’amidon comprenant les étapes suivantes :

   a) la liquéfaction de la matière contenant de l’amidon à l’aide d’une alpha-amylase ;

b) la saccharification de la matière liquéfiée à l'aide d'une glucoamylase ;

c) la fermentation à l'aide d'un organisme de fermentation, où la saccharification est effectuée en présence d'une xylanase GH5, la xylanase GH5 étant choisie dans la sous-famille 35.

**2.** Procédé selon la revendication 1, dans lequel la matière contenant de l'amidon comprend du maïs, du blé, du froment, du seigle, de l'orge, du triticale, du sorgho, du panic raide, du millet, du millet perlé, de la sétaire ou une forme transformée telle que du blé moulu, du froment moulu, du seigle moulu, de l'orge moulue, du triticale moulu, du maïs moulu, du maïs dégraissé, du maïs dégraissé désamidonné, du sorgho moulu, du panic raide moulu, du millet moulu, de la sétaire moulue, du millet perlé moulu.

**3.** Procédé selon l'une quelconque des revendications précédentes, dans lequel la matière contenant de l'amidon comprend du xylane fortement ramifié.

**4.** Procédé selon la revendication 1, dans lequel la xylanase GH5 de la sous-famille 35 est choisie parmi les xylanases représentées par les acides aminés 37 à 573 de la SEQ ID No: 2, les acides aminés 36 à 582 de la SEQ ID No: 4, ou une xylanase présentant une identité d'au moins 75 % avec les acides aminés 37 à 573 de la SEQ ID No: 2, ou les acides aminés 36 à 582 de la SEQ ID No: 4.

**5.** Procédé selon l'une quelconque des revendications précédentes, dans lequel la matière végétale comprend du blé, du sorgho, du froment, du seigle, de l'orge, ou du triticale.

**6.** Procédé selon l'une quelconque des revendications précédentes, dans lequel le produit de fermentation est de l'alcool, en particulier de l'éthanol.

**7.** Procédé selon l'une quelconque des revendications précédentes, dans lequel l'organisme de fermentation est une levure, en particulier *Saccharomyces* sp., plus particulièrement *Saccharomyces cerevisiae.*

**8.** Procédé selon l'une quelconque des revendications précédentes, dans lequel les étapes b) et c) sont effectuées simultanément.

**9.** Utilisation d'une xylanase GH5 de la sous-famille 35 pour produire de l'éthanol à partir d'une matière contenant de l'amidon.

**10.** Utilisation selon la revendication 9, dans laquelle la matière contenant de l'amidon comprend du blé, du sorgho, du froment, du seigle, de l'orge, ou du triticale.

# Figure 1

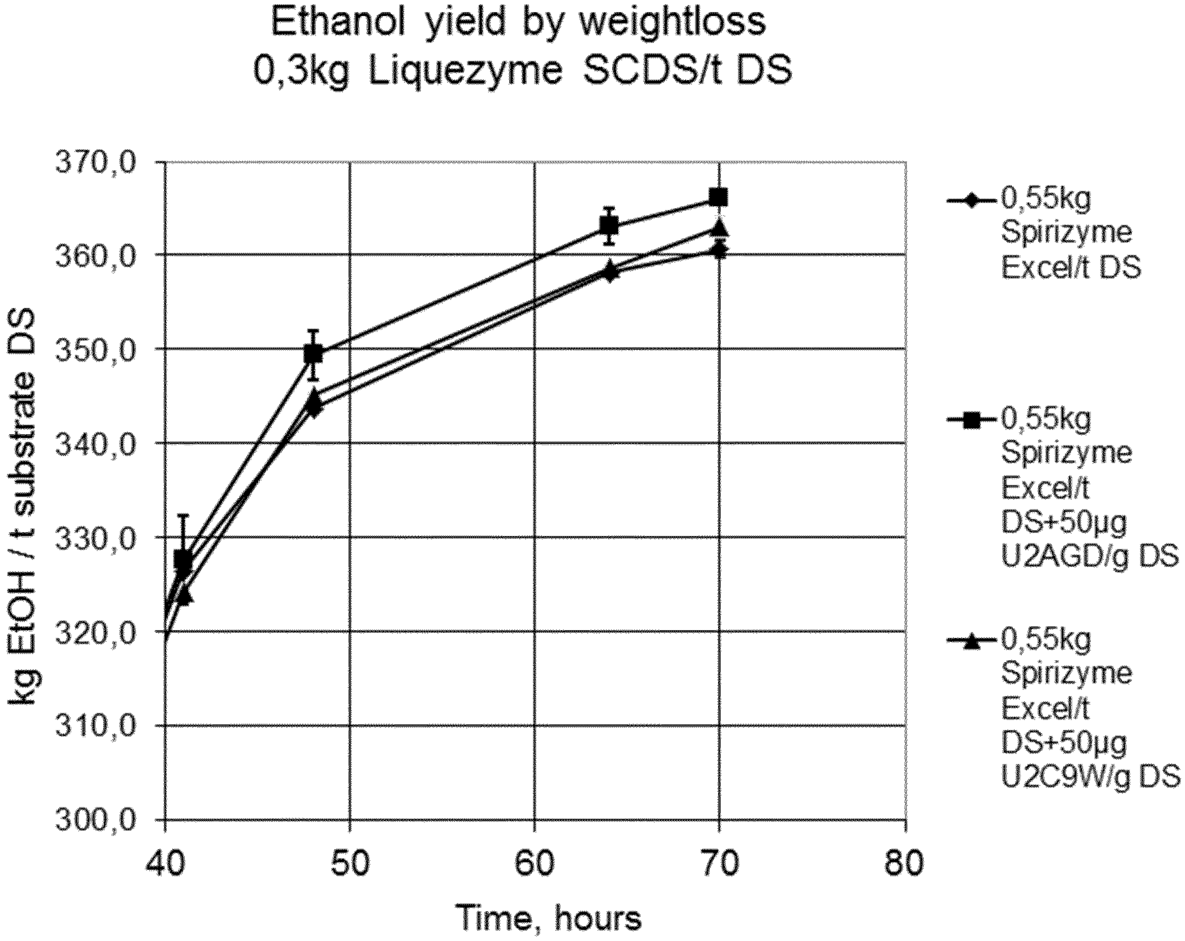
Ethanol yield by weightloss
0,3kg Liquezyme SCDS/t DS
kg EtOH / t substrate DS
370,0
360,0
350,0
340,0
330,0
320,0
310,0
300,0
40
50
60
70
80
Time, hours
0,55kg Spirizyme Excel/t DS
0,55kg Spirizyme Excel/t DS+50µg U2AGD/g DS
0,55kg Spirizyme Excel/t DS+50µg U2C9W/g DS

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9919467 A
- WO 12025577 A
- WO 9943835 A

### Non-patent literature cited in the description


- **POPPER ; TUOHY.** *Plant Physiology,* 2010, vol. 153, 373-383
- **HUISMANN et al.** *Carbohydrate Polymer,* 2000, vol. 42, 269-279
- **AGGER et al.** *J. Agric. Food Chem,* 2010, vol. 58, 6141-6148
- **HUISMANN et al.** *Carbohydrate Polymers,* 2000, vol. 42, 269-279
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **RICE et al.** *EMBOSS: The European Molecular Biology Open Software Suite,* 2000
- **ASPEBORG et al.** *BMC Evolutionary Biology,* 2012, vol. 12, 186
- **LOMBARD et al.** *Nucleic Acids Res,* 2014, vol. 42, D490-D495
- **HO et al.** *Applied and Environmental Microbiology,* 1998, 1852-1859
- **KARHUMAA et al.** *Microbial Cell Factories,* 2006, vol. 5, 18
- **LUCAS S. ; COPELAND A. ; LAPIDUS A. ; CHENG J.-F. ; BRUCE D. ; GOODWIN L. ; PITLUCK S. ; LAND M.L. ; HAUSER L. ; CHANG Y.-J.** *The draft genome of Acetivibrio cellulolyticus CD2,* August 2010
- **HEMME CL ; MOUTTAKI H ; LEE YJ ; ZHANG G ; GOODWIN L ; LUCAS S ; COPELAND A ; LAPIDUS A ; GLAVINA DEL RIO T ; TICE H et al.** Sequencing of multiple clostridial genomes related to biomass conversion and biofuel production. *J Bacteriol,* 2010, vol. 192 (24), 6494-6496
- **DIDERICHSEN et al.** *Plasmid,* 1993, vol. 30, 312-315
- **PETTERSSON et al.** Total dietary fiber determined as neutral sugar residues, uronic acid residues, and Klason lignin (the Uppsala method), Collaborative study. *J. AOAC Int.,* 1995, vol. 78, 1030-1044
- **CORREIA et al.** *J. Biol. Chem.,* 2011, vol. 286, 22510-22520